# EUROPÄISCHE PATENTSCHRIFT

(11) **EP 1 890 553 B1**
(45) Veröffentlichungstag und Bekanntmachung des Hinweises auf die Patenterteilung: **27.08.2008**
(21) Anmeldenummer: 06752579.0
(22) Anmeldetag: 14.06.2006
(51) Int. Cl.: A23K 1/00, A23K 1/18, A61K 35/74, A23L 1/30

(54) **PROBIOTISCHER, GESUNDHEITS- BZW. LEI STUNGS FÖRDERNDER NAHRUNGS-, FUTTERMITTEL UND/ODER TRINKWASSERZUSATZ SOWIE SEINE VERWENDUNG**
PROBIOTIC HEALTH OR FITNESS PROMOTING HUMAN OR ANIMAL FOODSTUFF AND/OR DRINKING WATER ADDITIVE AND USE THEREOF
PRODUIT ALIMENTAIRE, FOURRAGER ET/OU ADDITIF POUR EAU POTABLE PROBIOTIQUES, BONS POUR LA SANTE ET LA PERFORMANCE ET LEUR UTILISATION

(30) Priorität: 14.06.2005 AT 9992005
(43) Veröffentlichungstag der Anmeldung: 27.02.2008
(73) Patentinhaber: Erber Aktiengesellschaft, 3130 Herzogenburg (AT)
(72) Erfinder: PLAIL, Regina, A-3550 Langenlois (AT); SCHATZMAYR, Gerd, A-3430 Tulln (AT); BINDER, Eva, Maria, A-7501 Unterwart 269 (AT); MOHNL, Michaela, A-3430 Tulln (AT); KLIMITSCH, Alfred, A-3454 Reidling (AT); NITSCH, Sabine, A-3100 St. Pölten (AT); KLOSE, Viviana, A-1210 Wien (AT)
(74) Vertreter: Cunow, Gerda
(86) Internationale Anmeldenummer: PCT/AT2006/000243
(87) Internationale Veröffentlichungsnummer: WO 2006/133472

(56) Entgegenhaltungen:
- WO-A-99/19459
- WO-A-20/04032645
- DE-U1- 20 202 562

## Beschreibung

Die vorliegende Erfindung bezieht sich auf einen probiotischen, gesundheits- bzw. leistungsfördernden Nahrungs-, Futtermittel- und/oder Trinkwasserzusatz, welcher eine Mischung von Mikroorganismen enthält, sowie auf die Verwendung eines derartigen, probiotischen, gesundheits- bzw. leistungsfördernden Nahrungs-, Futtermittel- und/oder Trinkwasserzusatzes.

Nahrungs-, Futtermittel- und/oder Trinkwasserzusätze, welche eine Mischung von Mikroorganismen enthalten, werden verstärkt sowohl im humanen als auch tierischen Anwendungsbereich eingesetzt, um Infektionen durch pathogene Keime, wie beispielsweise Salmonellen oder E. coli, Campylobacter, Clostridien etc., möglichst hintanzuhalten. Der Einsatz von derartigen Mischungen von Mikroorganismen beruht auf der sogenannten "competitive exclusion" (CE), bei welcher versucht wird, mit sogenannten "guten" Bakterien die "schlechten oder bösen" Bakterien, d.h. gesundheitsschädigende bzw. -beeinträchtigende Bakterien, zu verdrängen bzw. auszuschließen. Hiebei hemmt das Wachstum der guten Bakterien jenes der bösen Bakterien, beispielsweise indem sie im Milieu des Verdauungstrakts einen Wachstumsvorteil haben und dadurch schneller proliferieren. Konkret wird dabei eine spezielle Mischung von Bakterien, welche auch aus dem Verdauungstrakt isoliert werden kann, eingesetzt, wobei versucht wird, möglichst breite Spektren an Darmbakterien zu verabreichen, um ein breites Anwendungsgebiet zu erzielen. Es hat sich jedoch der Einsatz von einem möglichst breiten Spektrum von Darmbakterien, welche nicht spezifiziert sind, als problematisch erwiesen, da einerseits etwaige Probleme undefinierter Mischungen, wie beispielsweise die Übertragung von Antibiotikaresistenzen oder Krankheiten, kaum vermieden werden können, und andererseits auch die Verabreichung von undefinierten Mischungen durch regionale oder internationale Regulierungen und Gesetzgebungen eingeschränkt ist, um nicht unerwartete bzw. unerwünschte Ergebnisse durch die Verabreichung derartiger Mischungen zu erzielen. Es wird daher auf den Einsatz definierter Mischungen, insbesondere auf definierte Probiotikakulturen, die aus einem oder mehreren Stämmen bestehen, zurückgegriffen werden müssen, um unerwartete Wirkungen zu vermeiden und der Gesetzgebung Genüge zu tun.

Unter Berücksichtigung von gesetzlichen Bestimmungen und in dem Wunsch, möglichst konkrete Ergebnisse zu erzielen, ist in letzter Zeit eine Vielzahl von Literaturstellen bekannt geworden, von welchen beispielsweise die US-A 5 372 810 eine Formulierung und ein Verfahren zur Prävention und Behandlung von Durchfall bei Nutztieren beschreibt, wobei sterilisierte Bakterienzellen, ihre Homogenate oder Zellwandbestandteile, die aerob gezüchtet wurden, eingesetzt werden. Die eingesetzten Bakterien gehören hiebei zum Genus Brevibacterium und/oder Corynebacterium.

Die US-Publikation 2004-0115308 beschreibt konsumierbare Produkte, die frische, probiotische Zutaten enthalten, wobei die Probiotika aus den Gruppen der Hefen, Aspergillen, Lactobacillen, Bifidobakterien, Streptococcen, Enterococcen und Mischungen davon bestehen. Die hier eingesetzten Bakterien sind nicht getrocknet oder in irgendeiner Weise aufkonzentriert, sondern werden wie gewonnen eingesetzt, wodurch Probleme in bezug auf die Haltbarkeit, Anwendbarkeit und dgl. entstehen.

Aus der EP 1 037 964 ist ein definiertes Probiotikum oder eine Formulierung von anaeroben Bakterien zur Kontrolle oder Inhibierung von Salmonellen bei Schweinen bekannt geworden, wobei die Bakterien Enterococcus faecalis, Streptococcus bovis, Clostridium clostridiforme, Clostridium symbiosum, Clostridium ramosum, Bacteroides fragilis, Bacteroides distasonis, Bacteroides vulgatus, Bacteroides thetaiotamicron und Bacteroides caccae umfassen und mindestens sieben verschiedene Gruppen von Keimen in dem Produkt enthalten sein müssen.

Schließlich beschreibt die WO 03/054168 ein Verfahren zur Herstellung einer Formulierung aus Darmkeimen, die die Fähigkeit besitzen, pathogene Keime auszuschließen. Bei diesem Verfahren wird eine Mikrobenprobe aus dem Darm einer bestimmten Tierspezies gewonnen und aeroben Bedingungen ausgesetzt, in der Folge eingefroren und wieder aufgetaut. Die einzelnen Mikrobengruppen werden selektiv kultiviert und in einem Inhibierungstest auf ihre Wirkung gegenüber pathogenen Mikroben getestet und schließlich fermentiert und eingesetzt.

Bei den in den Literaturstellen beschriebenen Verfahren bestehen jedoch einerseits Probleme dahingehend, daß nur eine Wirkung gegen einen speziellen pathogenen Keim erzielt werden kann, indem entweder einzelne spezifische Bakterien oder Kombinationen davon eingesetzt werden. Eine Wirkung gegenüber verschiedensten im Verdauungstrakt enthaltenen, pathogenen Keimen ist nicht erzielbar. Schließlich leiden die in der Literatur beschriebenen Verfahren meist auch daran, daß das verabreichte Spektrum an Bakterien nicht breit genug ist, um eine möglichst vollständige Inhibierung von pathogenen Keimen zu erzielen. Andere bekannte Produkte setzen nicht aus dem Darm gewonnene Bakterien ein, wodurch eine nicht vollständig spezifische Wirkung erzielt werden kann.

Die vorliegende Erfindung zielt nun darauf ab, einen probiotischen, gesundheits- bzw. leistungsfördernden Nahrungs-, Futtermittel- und/oder Trinkwasserzusatz, welcher eine Mischung von Mikroorganismen enthält, zur Verfügung zu stellen, welcher seine Wirkung im wesentlichen im gesamten Verdauungssystem von Säugern und/oder Nutzvögeln zeigt und dadurch insbesondere die schädliche Wirkung von einer Vielzahl von unerwünschten Keimen, wie beispielsweise Salmonellen, E. coli, Clostridien usw., verhindert oder zumindest reduziert.

Zur Lösung dieser Aufgaben wird gemäß der vorliegenden Erfindung ein probiotischer, gesundheits- bzw. leistungsfördernder Nahrungs-, Futtermittel- und/oder Trinkwasserzusatz, welcher eine Mischung von Mikroorganismen enthält, eingesetzt, in welchem wenigstens zwei Mikroorganismen, gewählt aus der Gruppe Enterococcus faecium, DSM 16211, Lactobacillus reuteri, DSM 16350, und Lactobacillus salivarius ssp. salivarius, DSM 16351, Pediococcus acidilactici, DSM 16210, und Bifidobacterium animalis, DSM 16284, enthalten sind. Dadurch, daß Mikroorganismen aus der Gruppe, bestehend aus Enterococcus faecium, DSM 16211, Lactobacillus reuteri, DSM 16350, und Lactobacillus salivarius ssp. salivarius, DSM 16351, Pediococcus acidilactici, DSM 16210, und Bifidobacterium animalis, DSM 16284, eingesetzt werden, gelingt es, aufgrund der Herkunft dieser Mikroorganismen aus den verschiedensten Bereichen des Verdauungstrakts, wie beispielsweise dem Blinddarm, Leerdarm, Ileum oder Kropf, im wesentlichen den gesamten Verdauungstrakt, insbesondere den Magen-Darm-Bereich, abzudecken, so daß pathogene Keime erfolgreich an ihrer Wirkung im gesamten Verdauungstrakt gehindert werden können.

Bei den hinterlegten Mikroorganismen handelt es sich mit der Ausnahme von DSM 16284 Bifidobacterium animalis, welcher eine neue Spezies darstellen dürfte, um neue Stämme bereits bekannter Spezies, da sie allgemein eine Übereinstimmung von über 99 % mit bekannten Stämmen zeigen.

Die einzelnen Sequenzen der hinterlegten Stämme lauten wie folgt:
Pediococcus acidilactici, DSM 16210 
CCTGGCTCAGGATGAACGCTGGCGGCGTGCCTAATACATGCAAGTCGAACGAACTTCCGT
TAATTGATTATGACGTGCTTGCACTGAATGAGATTTTAACACGAAGTGAGTGGCGGACGG
GTGAGTAACACGTGGGTAACCTGCCCAGAAGCAGGGGATAACACCTGGAAACAGATGCTA
ATACCGTATAACAGAGAAAACCGCCTGGTTTTCTTTTAAAAGATGGCTCTGCTATCACTT
CTGGATGGACCCGCGGCGCATTAGCTAGTTGGTGAGGTAACGGCTCACCAAGGCGATGAT
GCGTAGCCGACCTGAGAGGGTAATCGGCCACATTGGGACTGAGACACGGCCCAGACTCCT
ACGGGAGGCAGCAGTAGGGAATCTTCCACAATGGACGCAAGTCTGATGGTGCACGCCGCG
TGAGTGAAGAAGGGTTTCGGCTCGTAAAGCTCTGTTGTTAAAGAAGAACGTGGGTGAGAG
TAACTGTTCACCCAGTGACGGTATTTAACCAGAAAGCCACGGCTAACTACGTGCCAGCAG
CCGCGGTAATACGTAGGTGGCAAGCGTTATCCGGATTTATTGGGCGTAAAGCGAGCGCAG
GCGGTCTTTTAAGTCTAATGTGAAAGCCTTCGGCTCAACCGAAGAAGTGCATTGGAAACT
GGGAGACTTGAGTGCAGAAGAGGATAGTGGAACTCCATGTGTAGCGGTGAAATGCGTAGA
TATATGGAAGAACACCAGTGGCGAAGGCGGCTGTCTGGTCTGTAACTGACGCTGAGGCTC
GAAAGCATGGGTAGCGAACAGGATTAGATACCCTGGTAGTCCATGCCGTAAACGATGATT
ACTAAGTGTTGGAGGGTTTCCGCCCTTCAGTGCTGCAGCTAACGCATTAAGTAATCCGCC
TGGGGAGTACGACCGCAAGGTTGAAACTCAAAAGAATTGACGGGGGCCCGCACAAGCGGT
GGAGCATGTGGTTTAATTCGAAGCTACGCGAAGAACCTTACCAGGTCTTGACATCTTCTG
CCAACCTAAGAGATTAGGCGTTCCCTTCGGGGACAGAATGACAGGTGGTGCATGGTTGTC
GTCAGCTCGTGTCG
Sequenzierungsprimer: 341 forward, 530 reverse Contig aus 2 Partialsequenzen: 341f530r
Sequenzlänge: 1094 Basen insgesamt
   Übereinstimmung mit einem bekannten Stamm 99,6 %
Lactobacillus reuteri, DSM 16350 
GGATGAACGCCGGCGGTGTGCCTAATACATGCAAGTCGTACGCACTGGCCCAACTGATTG
ATGGTGCTTGCACCTGATTGACGATGGATCACCAGTGAGTGGCGGACGGGTGAGTAACAC
GTAGGTAACCTGCCCCGGAGCGGGGGATAACATTTGGAAACAGATGCTAATACCGCATAA
CAACAAAAGCCACATGGCTTTTGTTTGAAAGATGGCTTTGGCTATCACTCTGGGATGGAC
CTGCGGTGCATTAGCTAGTTGGTAAGGTAACGGCTTACCAAGGCGATGATGCATAGCCGA
GTTGAGAGACTGATCGGCCACAATGGAACTGAGACACGGTCCATACTCCTACGGGAGGCA
GCAGTAGGGAATCTTCCACAATGGGCGCAAGCCTGATGGAGCACACCGCGTGAGTGAAGA
AGGGTTTCGGCTCGTAAAGCTCTGTTGTTGGAGAAGAACGTGCGTGAGAGTAACTGTTCA
YGCAGTGACGGTATCCAACCAGAAAGTCACGGCTAACTACGTGCCAGCAGCCGCGGTAAT
ACGTAGGTGGCAAGCGTTATCCGGATTTATTGGGCGTAAAGCGAGCGCAGGCGGTTGCTT
AGGTCTGATGTGAAAGCCTTCGGCTTAACCGAAGAAGTGCATCGGAAACCGGGCGACTTG
AGTGCAGAAGAGGACAGTGGAACTCCATGTGTAGCGGTGGAATGCGTAGATATATGGAAG
AACACCAGTGGCGAAGGCGGCTGTCTGGTCTGCAACTGACGCTGAGGCTCGAAAGCATGG
GTAGCGAACAGGATTAGATACCCTGGTAGTCCATGCCGTAAACGATGAGTGCTAGGGTGT
TGGAGGGTTTCCGCCCTTCAGTGCCGGAGCTAACGCATTAAGCACTCCGCCTGGGGAGTA
CGACCGCAAGGTTGAAACTCAAAGGAATTGACGGGGGCCCGCACAAGCGGTGGAAGCATG
TGGTTTAATTCGAAGCTACGCGAAGAACCTTACCAGGTCTTGACATCTTGCGCTAACCTT
AGAGATAAGGCGTTCCCTTCGGGGACGCAATGACAGGTGGTGCATG
GTCGTCGTCAGCTCGTG
Sequenzierungsprimer: 341 forward, 530 reverse Contig aus 2 Partialsequenzen: 341f530r
Sequenzlänge: 1083 Basen insgesamt
   Übereinstimmung mit einem bekannten Stamm 99,7 %
Lactobacillus salivarius ssp. salivarius, DSM 16351 
ACGCTGGCGGCGTGCCTAATACATGCAAGTCGAACGAAACTTTCTTACACCGAATGCTTG
CATTCACTCGTAAGAAGTTGAGTGGCGGACGGGTGAGTAACACGTGGGTAACCTGCCTAA
AAGAAGGGGATAACACTTGGAAACAGGTGCTAATACCGTATATCTNTAAGGATCGCATGA
TCCTNAGATGAAAGATGGTTCTGCTATCGCTTNTAGATGGACCCGCGGCGTATTANCTAG
TTGGTGGGGTAACGGCNTACCAAGGNGATGATACGTAGCCGAACTGAGAGGNTGATCGGC
CACATTGGGACTGAGACACGGCCCAAACTCCTACGGGAGGCAGCAGTAGGGAATCTTCCA
CAATGGACGCAAGTCTGATGGTGCCCGCCGCGAGAGTGAAGAAGGTCTTCGGATCGTAAA
ACTCTGTTGTTAGAGAAGAACACGAGTGAGAGTAACTGTTCATTCGATGACGGTATCTAA
CCAGCAAGTCACGGCTAACTACGTGCCAGCAGCCGCGGTAATACGTAGGTGGCAAGCGTT
GTCCGGATTTATTGGGCGTAAAGGGAACGCAGGCGGTCTTTTAAGTCTGATGTGAAAGCC
TTCGGCTTAACCGGAGTAGTGCATTGGAAACTGGAAGACTTGAGTGCAGAAGAGGAGAGT
GGAACTCCATGTGTAGCGGTGAAATGCGTAGATATATGGAAGAACACCAGTGGCGAAAGC
GGCTCTCTGGTCTGTAACTGACGCTGAGGTTCGAAAGCGTGGGTAGCAAACAGGATTAGA
TACCCTGGTAGTCCACGCCGTAAACGATGAATGCTAGGTGTTGGAGGGTTTCCGCCCTTC
AGTGCCGCAGCTAACGCAATAAGCATTCCGCCTGGGGAGTACGACCGCAAGGTTGAAACT
CAAAGGAATTGACGGGGGCCCGCACAAGCGGTGGAGCATGTGGTTTAATTCGAAGCAACG
CGAAGAACCTTACCAGGTCTTGACATCCTTTGACCACCTAAAAATTAGGTTTCCCTTCG
 GGGACAAAGTGACAGGTGGTGCATGGCTGTCGTCAGCTCGTGTCGT
Sequenzierungsprimer: 341 forward, 530 reverse
Contig aus 2 Partialsequenzen: 341f530r
Sequenzlänge: 1066 Basen insgesamt
   Übereinstimmung mit einem bekannten Stamm 99,5 %
Enterococcus faecium, DSM 16211 
CCTGGCTCAGGACGAACGCTGGCGGCGTGCCTAATACATGCAAGTCGAACGCTTCTTTT
TCCACCGGAGCTTGCTCCACCGGAAAAAGAGGAGTGGCGAACGGGTGAGTAACACGTGG
GTAACCTGCCCATCAGAAGGGGATAACACTTG
GAAACAGGTGCTAATACCGTATAACAATCAAAACCGCATGGTTTTGATTTGAAAGGCGC
TTTCGGGTGTCGCTGATGGATGGACCCGCGGTGCATTAGCTAGTTGGTGAGGTAACGGC
TCACCAAGGCCACGATGCATAGCCGACCTGAG
AGGGTGATCGGCCACATTGGGACTGAGACACGGCCCAAACTCCTACGGGAGGCAGCAGT
AGGGAATCTTCGGCAATGGACGAAAGTCTGACCGAGCAACGCCGCGTGAGTGAAGAAGG
TTTTCGGATCGTAAAACTCTGTTGTTAGAGAA
GAACAAGGATGAGAGTAACTGTTCATCCCTTGACGGTATCTAACCAGAAAGCCACGGCT
AACTACGTGCCAGCAGCCGCGGTAATACGTAGGTGGCAAGCGTTGTCCGGATTTATTGG
GCGTAAAGCGAGCGCAGGCGGTTTCTTAAGTCTGATGTGAAAGCCCCCGGCTCAACCGG
GGAGGGTCATTGGAAACTGGGAGACTTGAGTGCAGAAGAGGAGAGTGGAATTCCATGTG
TAGCGGTGAAATGCGTAGATATATGGAGGAACACCAGTGGCGAAGGCGGCTCTCTGGTC
TGTAACTGACGCTGAGGCTCGAAAGCGTGGGGAGCAAACAGGATTAGATACCCTGGTAG
TCCACGCCGTAAACGATGAGTGCTAAGTGTTGGAGGGTTTCCGCCCTTCAGTGCTGCAG
CTAACGCATTAAGCACTCCGCCTGGGGAGTACGACCGCAAGGTTGAAACTCAAAGGAAT
TGACGGGGGCCCGCACAAGCGGTGGAGCATGTGGTTTAATTCGAAGCAACGCGAAGAAC
CTTACCAGGTCTTGACATCCTTTGACCACTCTAGAGATAGAGCTTCCCCTTC
Sequenzierungsprimer: 341 forward, 530 reverse
Contig aus 2 Partialsequenzen: 341f530r
Sequenzlänge: 1033 Basen insgesamt
   Übereinstimmung mit einem bekannten Stamm 99,9 %
Bifidobacterium animalis, DSM 16284 
TTGCCATGGGCGCAAGCCTGATGCAGCGACGCCGCGTGCGGGATGGAGGCCTTCGGGTTG
TAAACCGCTTTTGTTCAAGGGCAAGGCACGGTTTCGGGCCGTGTTGAGTGGATTGTTCGA
ATAAGCACCGGCTAACTACGTGCCAGCAGCCGCGGTAATACGTAGGGTGCGAGCGTTATC
CGGATTTATTGGGCGTAAAGGGCTCGTAGGCGGTTCGTCGCGTCCGGTGTGAAAGTCCAT
CGCCTAACGGTGGATCTGCGCCGGGTACGGGCGGGCTGGAGTGCGNTAAGGAGACTGGA
ATTCCCGGTGTAACGNTGGAATGTGTANATATCGGGAAGAACACCAATGGNNAANGNAGG
TCTCTGGGCCGTTACTGACGCTGACGATNNAAAGACGTGAACCAGCGANCNCNATAANAT
ACCCTGACTACGGATTAGATACCCTGGTAGTCCACGCCGTAAACGGTGGATGCTGGATGT
 
GGGGCCCTTTCCACGGGTCCTGTGTCGGAGCCAACGCGTTAAGCATCCCGCCTGGGGAGT
ACGGCCGCAAGGCTAAAACTCAAAGAAATTGACGGGGGCCCGCACAAGCGGCGGAGCATG
CGGATTAATTCGATGCAACGCGAAGAACCTTACCTGGGCTTGACATGTGCCGGATCGCCG
TGGAAACACGGTTTCCCTTCGGGGCCGGTTCACAGGTGGTGCATGGTCGTCGTCAGCTCG
TGTCGTGAGATGTTGGGTTAAGTCCCGCAACGAGCGCAACCCTCGCCGCATGTTGCCAGC
GGGTGATGCCGGGAACTCATGTGGACCGCCGGGGTCAACTCGGAGGAAGGTGGGGATGA
CGTCAGATCATCATGCCCCTTACGTCCAGGGCTTCACGCATGCTACAATGGCCGGTACAA
CGCGATGCGACACGGTGACGTGGGGCGGATCGCTGAAAACCGGTCTCAGTTCGGATCGCA
GTCTGCAACTCGACTGCGTGAAGGCGGAGTCGCTAGTAATCGCGGATCAGCAACGCCGCG
GTGAATGCGTTCCCGGGCCTTGTACACACCGCCCGTCAAGTCATGAAAGTGGGTAGCACC
GGAAGCCGGTGGCCCGACCCTCGTGGGGCGGACCGTCTAATGGTGAGACTCGTGATTGG
Sequenzierungsprimer: 341 forward, 1492 reverse
Contig aus 2 Partialsequenzen: 341f1492r
Sequenzlänge: 1139 Basen insgesamt
   Übereinstimmung mit einem bekannten Stamm 98,9 % - neue Spezies

Gemäß einer Weiterbildung der Erfindung sind in dem Nahrungs-, Futtermittel- und/oder Trinkwasserzusatz wenigstens drei Mikroorganismen enthalten, wodurch eine vollständigere Abdeckung des gesamten Verdauungstrakts erzielt werden kann und darüber hinaus auch eine synergistische Wirkung der drei eingesetzten Mikroorganismen gegenüber pathogenen Keimen nachgewiesen werden kann.

Als besonders bevorzugte Mischung für Nahrungs-, Futtermittel- und/oder Trinkwasserzusätze hat sich hiebei eine Mischung erwiesen, in welcher Enterococcus faecium, DSM 16211, Lactobacillus reuteri, DSM 16350, und Lactobacillus salivarius ssp. salivarius, DSM 16351, enthalten ist. Eine derartige Mischung hat sich nicht nur bei der Inhibierung von pathogenen Keimen, insbesondere E. coli Bakterien als günstig erwiesen, sondern es wird auch aufgrund ihres starken pH-Absenkungspotentials und der hohen Produktionsraten von Säure, beispielsweise Milchsäure, das Lebensumfeld für pathogene Keime stark eingeschränkt, wodurch auch die Ansiedlung von weiteren, anderen Pathogenen zusätzlich stark inhibiert werden kann.

Weiters besonders bevorzugt ist ein Nahrungs-, Futtermittel- und/oder Trinkwasserzusatz, in welchem Enterococcus faecium, DSM 16211, Pediococcus acidilactici, DSM 16210, und Bifidobacterium animalis, DSM 16284, enthalten sind. Ein derartiger Nahrungs-, Futtermittel- und/oder Trinkwasserzusatz zeigt insbesondere eine gleichmäßig verbesserte Inhibierung von nahezu sämtlichen, üblicherweise vorhandenen, pathogenen Keimen, insbesondere E. coli, Salmonella choleraesuis, Campylobacter jejuni, Clostridium perfringens, welche gegenüber der Einzelwirkung der in der Mischung enthaltenen Mikroorganismen derartig stark erhöht ist, daß eine synergistische Wirkung der drei Mikroorganismen gegenüber einer Vielzahl von pathogenen Keimen erzielbar ist. Darüber hinaus zeigt auch diese Mischung ein starkes pH-Absenkungspotential, insbesondere aufgrund der relativ hohen Mengen an Milchsäure und Essigsäure, welche gebildet werden, wodurch wiederum die Lebensbedingungen für pathogene Keime stark nachteilig beeinflußt werden.

Indem, wie dies einer bevorzugten Weiterbildung der vorliegenden Erfindung entspricht, in dem Nahrungs-, Futtermittel- und/oder Trinkwasserzusatz zusätzlich als Träger einsetzbare Nährsubstanzen und/oder prebiotische Substanzen, gewählt aus Fructo-Oligosacchariden, Inulinen, Isomalto-Oligosacchariden, Lactitol, Lactosucrose, Lactulose, Pyrodextrinen, Soja-Oligosacchariden, Transgalacto-Oligosacchariden, Xylo-Oligosaccardien, Vitaminen, insbesondere Vitamin E, enthalten sind, werden die Lebensbedingungen der eingesetzten Mikroorganismen im Verdauungstrakt deutlich verbessert und es wird insbesondere dafür Sorge getragen, daß sich die eingesetzten Mikroorganismen im Verdauungstrakt rasch und zuverlässig vermehren können und gleichzeitig jedoch die Vermehrung von pathogenen Keimen durch "competitive exclusion" hintangehalten bzw. verhindert wird. Aufgrund der eingesetzten Nährsubstanzen und/oder prebiotischen Säuren wird ein Wachstumsvorteil für die eingesetzten Mikroorganismen gegenüber pathogenen Keimen geschaffen.

Indem, wie dies einer bevorzugten Weiterbildung der vorliegenden Erfindung entspricht, ein weiterer Träger, gewählt aus Zeolithen, Calciumkarbonat, Magnesiumkarbonat, Trehalose, Chitosan, Schellack, Albumin, Stärke, Magermilchpulver, Süßmolkenpulver, Maltodextrine, Lactose, Inulin, Dextrosen, pflanzlichen Ölen, oder ein Lösungsmittel, gewählt aus Wasser oder physiologischer Kochsalzlösung, enthalten ist, kann einerseits eine gleichmäßige Verteilung der Mikroorganismen im Verdauungstrakt erzielt werden und andererseits eine weitere Unterstützung der "competitive exclusion" erzielt werden.

Indem, wie dies einer Weiterbildung der Erfindung entspricht, zusätzlich ein Beschichtungsmaterial, gewählt aus Maltodextrinen, Guarkernmehl, Gummi arabicum, Alginaten, modifizierter Stärke und Stärkederivaten, Dextrinen, Cellulosederivaten, wie Celluloseester und -ether, Proteinen, wie Gelatine, Albumin, Casein, Gluten, Akaziengummi, Traganthgummi, Lipiden, wie Wachsen, Paraffin, Stearinsäure, Mono- und Diglyceriden, enthalten ist, gelingt es, die Mikroorganismen gemeinsam mit gegebenenfalls enthaltenen Trägermaterialien zu beschichten, wodurch sichergestellt wird, daß die Mikroorganismen erst am Ort ihres Einsatzzwecks, d.h. im Verdauungstrakt, ihre Wirkung entfalten können. Durch Einsatz derartiger Beschichtungsmaterialien ist es darüber hinaus beispielsweise möglich, den Nahrungs-, Futtermittel- und/oder Trinkwasserzusatz direkt auf damit zu behandelnde Tiere, wie einen Tag alte Küken, in ihrer Transportbox durch sogenannte Gel- oder Granulatapplikationen aufzubringen, wodurch eine Prophylaxe und eine therapeutische Behandlung von Jungtieren gegenüber Infektionen durch pathogene Mikroben zur Verfügung gestellt wird. Derartige Anwendungen sind in der modernen Tierzucht insbesondere von Bedeutung, da aufgrund des fehlenden, direkten Kontakts von Jungtieren mit den Muttertieren eine entsprechende Weitergabe einer Grundlage für eine intakte Darmflora nicht mehr gegeben ist.

Um eine noch vollständigere Wirkung des Nahrungs-, Futtermittel- und/oder Trinkwasserzusatzes gegenüber pathogenen Keimen zu erzielen, ist der Zusatz bevorzugt dahingehend weitergebildet, daß zusätzlich wenigstens ein Mikroorganismus, gewählt aus der Gruppe Bifidobacterium sp., Lactobacillus salivarius, Lactobacillus sp., Lactobacillus fermentum und Enterococcus faecalis enthalten ist. Indem ein weiterer, an sich bekannter Mikroorganismus dem Nahrungs-, Futtermittel- und/oder Trinkwasserzusatz beigemischt ist, kann gezielt die Wirkung gegenüber einem oder mehreren bei den jeweiligen Tieren zu erwartenden, pathogenen Keim(en) verstärkt werden, wodurch eine weitere Absenkung der Infektionsgefahr erzielt werden kann.

Gemäß einer bevorzugten Weiterbildung hat es sich insbesondere als günstig erwiesen, den Nahrungs-, Futtermittel- und/oder Trinkwasserzusatz in suspendierter, pulverförmiger oder verkapselter Form mit einem maximalen Durchmesser von 2000 µm zum Einsatz zu bringen, wodurch je nach gewünschtem Einsatzort, wie beispielsweise Kropf, Magen, Dünndarm und dgl., eine exakte, gezielte Freisetzung der Wirkung der Mikroorganismen erzielbar ist. Darüber hinaus können selbstverständlich auch Mischformen aus suspendiertem und verkapseltem Nahrungs-, Futtermittel- und/oder Trinkwasserzusatz zum Einsatz gebracht werden, um die Wirkung über das gesamte Verdauungssystem sicherstellen zu können.

Gemäß einer bevorzugten Weiterbildung der Erfindung wird ein Futtermittelzusatz, insbesondere für Nutzvögel und/oder Schweine, zur Leistungssteigerung der Nutztiere zur Verfügung gestellt, welcher aus 1 x 10⁷ bis 1 x 10¹⁴, insbesondere 1 x 10¹⁰ bis 1 x 10¹², KBE/kg Futtermittelzusatz Enterococcus faecium, DSM 16211, und 1 x 10⁶ bis 1 x 10¹⁴, insbesondere 1 x 10¹⁰ bis 1 x 10¹², KBE/kg Futtermittelzusatz Lactobacillus reuteri, DSM 16350, besteht. Durch eine derartige Auswahl der Mengen an eingesetzten Mikroorganismen gelingt es insbesondere, einen Futtermittelzusatz herzustellen, welcher eine exzellente Wirkung gegenüber den meisten E. coli Keimen zeigt.

Gemäß einer bevorzugten Weiterbildung der Erfindung wird ein Futtermittelzusatz, insbesondere für Nutzvögel und/oder Schweine, zur Leistungssteigerung der Nutztiere zur Verfügung gestellt, welcher aus 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁷ bis 1 x 10¹⁴, insbesondere 1 x 10¹⁰ bis 1 x 10¹², KBE/kg Futtermittelzusatz Pediococcus acidilactici, DSM 16210, und 1 x 10⁷ bis 1 x 10¹⁴, insbesondere 1 x 10¹⁰ bis 1 x 10¹², KBE/kg Futtermittelzusatz Bifidobacterium animalis, DSM 16284, besteht. Mit einem derartigen Futtermittelzusatz gelingt es insbesondere, die Wirkung von Salmonella choleraesuis bzw. Clostridium perfringens ebenso wie die Wirkung von E. coli Bakterien stark bzw. gleichmäßig stark zu inhibieren und darüber hinaus aufgrund der hohen Produktion von Säuren, nämlich Essigsäure und Milchsäure, der eingesetzten Mikroorganismen den pH-Wert im Verdauungstrakt der damit gefütterten Tiere derart stark abzusenken, daß weitere pathogene Keime an ihrem Wachstum stark behindert sind.

Zur Verstärkung der Wirkung des Futtermittelzusatzes ist er bevorzugt dahingehend weitergebildet, daß er aus 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁶ bis 1 x 10¹⁴, insbesondere 1 x 10¹⁰ bis 1 x 10¹², KBE/kg Futtermittelzusatz Lactobacillus reuteri, DSM 16350, und 1 x 10⁶ bis 1 x 10¹⁴, insbesondere 1 x 10¹⁰ bis 1 x 10¹², KBE/kg Futtermittelzusatz Lactobacillus salivarius ssp. salivarius, DSM 16351, besteht. Durch eine derartige Auswahl der Mengen der eingesetzten Mikroorganismen gelingt es insbesondere, einen Futtermittelzusatz herzustellen, welcher eine exzellente Wirkung gegenüber E. coli Keimen zeigt und darüber hinaus aufgrund des hohen pH-Absenkungspotentials eine starke Inhibierung des Wachstums von nahezu sämtlichen weiteren pathogenen Keimen ermöglicht.

Der Futtermittelzusatz, insbesondere für Nutzvögel und/oder Schweine zur Leistungssteigerung der Nutztiere, besteht gemäß einer bevorzugten Weiterbildung der vorliegenden Erfindung aus 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁶ bis 1 x 10¹⁴, insbesondere 1 x 10¹⁰ bis 1 x 10¹², KBE/kg Futtermittelzusatz Lactobacillus reuteri, DSM 16350, 1 x 10⁶ bis 1 x 10¹⁴, insbesondere 1 x 10¹⁰ bis 1 x 10¹², KBE/kg Futtermittelzusatz Lactobacillus salivarius ssp. salivarius, DSM 16351, 1 x 10⁷ bis 1 x 10¹⁴, insbesondere 1 x 10¹⁰ bis 1 x 10¹², KBE/kg Futtermittelzusatz Pediococcus acidilactici, DSM 16210, 1 x 10⁷ bis 1 x 10¹⁴, insbesondere 1 x 10¹⁰ bis 1 x 10¹², KBE/kg Futtermittelzusatz Bifidobacterium animalis, DSM 16284, und 1 x 10⁶ bis 1 x 10¹⁴, insbesondere 1 x 10¹⁰ bis 1 x 10¹² KBE/kg Futtermittelzusatz Bifidobacterium sp. 2. Eine derartige Mischung des Futtermittelzusatzes zeigt insbesondere eine deutlich erhöhte Wirkung gegenüber den pathogenen Keimen Campylobacter jejuni ebenso wie Salmonella choleraesuis. Des weiteren sind auch die Wirkungen gegenüber gängigen E. coli Bakterien stark verbessert und gehen insgesamt weit über die Wirkung der Einzelstämme hinaus. Des weiteren zeigt die Stoffwechselreaktion eines derartigen Futtermittelzusatzes, daß eine synergistische Wirkung der Bakterien vorhanden sein muß. So kann die vorliegende Mischung aus fünf Mikroorganismen unter anderem Xylitol verwerten, was kein Mikroorganismus der Mischung alleine kann. Dies läßt sich damit begründen, daß für den Abbau von Xylitol beispielsweise mehrere Enzyme notwendig sind, und lediglich durch die erfindungsgemäße Kombination von fünf Mikroorganismen die nötigen Enzyme gemeinsam zur Verfügung gestellt werden können, wodurch ein derartig kompliziert abzubauendes Kohlehydrat verwertet werden kann.

Gemäß einer bevorzugten Weiterbildung enthält der Futtermittelzusatz zusätzlich 1 % - 95 %, insbesondere 20 % - 98 %, eines Trägers bzw. Naturstoffes. Dadurch, daß zusätzlich ein Träger bzw. ein Naturstoff enthalten ist, gelingt eine weitere Verdrängung von pathogenen Keimen aus dem Magen-Darm-Milieu, indem das gezielte Wachstum der eingesetzten Mikroorganismen unterstützt bzw. gefordert wird.

Indem, wie dies einer bevorzugten Weiterbildung der Erfindung entspricht, die wäßrige Bakteriensuspension in einer Menge von 0,01 g/kg - 10 g/kg Futtermittel, insbesondere 0,025 g/kg bis 2,5 g/kg Futtermittel, auf das Futtermittel bzw. dessen Pellets aufgebracht ist, wird einerseits sichergestellt, daß der Futtermittelzusatz problemlos von den Tieren aufgenommen wird und andererseits bereits vor Aufnahme des Futtermittels auf der Oberfläche des Futtermittels vorhandene, pathogene Keime durch den Futtermittelzusatz bekämpft bzw. verdrängt oder abgebaut werden können. Um eine besonders gute Wirkung des Futtermittelzusatzes zu erzielen, wird der Futtermittelzusatz bevorzugt in einer Menge von 20g/t bis 20 kg/t, insbesondere 100 g/t bis 2,5 kg/t in das Futtermittel eingemischt, wodurch einerseits eine ausreichende Menge an Mikroorganismen bereitgestellt ist und andererseits ein sicherer Abbau bzw. eine sichere Verdrängung von pathogenen Keimen aus dem Magen-Darm-Milieu sichergestellt werden kann.

Gemäß einer bevorzugten Weiterbildung der Erfindung werden die Mikroorganismen bzw. die Mischung aus Mikroorganismen als Trinkwasserzusatz zum Einsatz gebracht, wobei gemäß einer bevorzugten Weiterbildung der Trinkwasserzusatz 1 x 10⁷ bis 1 x 10¹⁴, insbesondere 1 x 10¹⁰ bis 1 x 10¹³, KBE/kg Trinkwasserzusatz Enterococcus faecium, DSM 16211, und 1 x 10⁶ bis 1 x 10¹⁴, insbesondere 1 x 10¹⁰ bis 1 x 10¹³, KBE/kg Trinkwasserzusatz Lactobacillus reuteri, DSM 16350, enthält. Ein derartiger Trinkwasserzusatz ist insbesondere bei der Inhibierung von pathogenen Keimen und hiebei insbesondere bei der Inhibierung von E. coli besonders günstig. Darüber hinaus kann eine derartige Mischung aufgrund der Bildung von Milchsäure, Essigsäure und Proprionsäure den pH-Wert im Magen-Darm-Trakt stark absenken, wodurch ungünstige Lebensbedingungen für pathogene Keime geschaffen werden und andererseits die gebildeten Säuren, wie dies allgemein bekannt ist, in ihrer nicht dissoziierten Form auch bakterizid wirken, so daß eine insgesamt gute Wirkung gegen eine Vielzahl von pathogenen Keimen erzielt werden kann.

Gemäß einer bevorzugten Weiterbildung wird ein Trinkwasserzusatz eingesetzt, welcher aus 1 x 10⁷ bis 1 x 10¹⁴, insbesondere 1 x 10¹⁰ bis 1 x 10¹³, KBE/kg Trinkwasserzusatz Enterococcus faecium, DSM 16211, 1 x 10⁶ bis 1 x 10¹⁴, insbesondere 1 x 10¹⁰ bis 1 x 10¹³, KBE/kg Trinkwasserzusatz Lactobacillus reuteri, DSM 16350, und 1 x 10⁶ bis 1 x 10¹⁴, insbesondere 1 x 10¹⁰ bis 1 x 10¹³, KBE/kg Trinkwasserzusatz Lactobacillus salivarius ssp. salivarius, DSM 16351, besteht. Ein derartiger Trinkwasserzusatz hat sich insbesondere bei der Inhibierung von E. coli sowie Salmonella choleraesuis als günstig erwiesen und zeigt darüber hinaus auch eine synergistische Wirkung in bezug auf das Stoffwechselprofil. So kann diese Mischung beispielsweise D-Tagatose abbauen, was keiner der enthaltenen Einzelstämme für sich gesehen kann. Es ist damit eine synergistische Wirkung dieser drei Stämme in bezug auf das Stoffwechselprofil nachweisbar.

Gemäß einer bevorzugten Weiterbildung ist die Erfindung dahingehend weitergebildet, daß der Trinkwasserzusatz aus 1 x 10⁷ bis 1 x 10¹⁴, insbesondere 1 x 10¹⁰ bis 1 x 10¹³, KBE/kg Trinkwasserzusatz Enterococcus faecium, DSM 16211, 1 x 10⁷ bis 1 x 10¹⁴, insbesondere 1 x 10¹⁰ bis 1 x 10¹³, KBE/kg Trinkwasserzusatz Pediococcus acidilactici, DSM 16210, und 1 x 10⁷ bis 1 x 10¹⁴, insbesondere 1 x 10¹⁰ bis 1 x 10¹³, KBE/kg Trinkwasserzusatz Bifidobacterium animalis, DSM 16284, besteht. Eine derartige Mischung von Mikroorganismen hat sich insbesondere bei der Inhibierung von pathogenen Keimen aus der Gruppe Salmonella choleraesuis, Campylobacter jejuni und Clostridium perfringens als günstig erwiesen und ist darüber hinaus aufgrund der unterschiedlichen Herkunft der Mikroorganismen aus dem Magen-Darm-Trakt in der Lage, auch die Wirkung über den gesamten Magen-Darm-Trakt zu entfalten, wodurch eine nahezu vollständige Verdrängung von schädlichen, pathogenen Keimen im gesamten Verdauungssystem erzielt werden kann.

Gemäß einer bevorzugten Weiterbildung wird ein Trinkwasserzusatz zum Einsatz gebracht, bestehend aus 1 x 10⁷ bis 1 x 10¹⁴, insbesondere 1 x 10¹⁰ bis 1 x 10¹³, KBE/kg Trinkwasserzusatz Enterococcus faecium, DSM 16211, 1 x 10⁶ bis 1 x 10¹⁴, insbesondere 1 x 10¹⁰ bis 1 x 10¹³, KBE/kg Trinkwasserzusatz Lactobacillus reuteri, DSM 16350, 1 x 10⁶ bis 1 x 10¹⁴, insbesondere 1 x 10¹⁰ bis 1 x 10¹³, KBE/kg Trinkwasserzusatz Lactobacillus salivarius ssp. salivarius, DSM 16351, 1 x 10⁷ bis 1 x 10¹⁴, insbesondere 1 x 10¹⁰ bis 1 x 10¹³, KBE/kg Trinkwasserzusatz Pediococcus acidilactici, DSM 16210, 1 x 10⁷ bis 1 x 10¹⁴, insbesondere 1 x 10¹⁰ bis 1 x 10¹³, KBE/kg Trinkwasserzusatz Bifidobacterium animalis, DSM 16284, und 1 x 10⁶ bis 1 x 10¹⁴, insbesondere 1 x 10¹⁰ bis 1 x 10¹³ KBE/kg Trinkwasserzusatz Bifidobacterium sp. Mit einem derartigen Trinkwasserzusatz gelingt es, nahezu sämtliche, häufig vorkommende, pathogene Keime, insbesondere E. coli, Salmonellen, Clostridien und Campylobacter, in einem Ausmaß zu inhibieren, welches weit über Summenwirkung der einzelnen Mikroorganismen hinausgeht, so daß eine massive synergistische Wirkung gegenüber pathogenen Keimen erzielt werden kann. Darüber hinaus gelingt es mit einem derartigen Mikroorganismus, den pH-Wert im Verdauungstrakt von Tieren stark und überaus rasch abzusenken, wodurch die Wirkung und insbesondere die Inhibierung des Wachstums von weiteren pathogenen Keimen weiter verstärkt wird.

Um insbesondere das Wachstum der erwünschten Mikroorganismen im Magen-Darm-Trakt zu fördern und die Verdrängungswirkung noch zu verstärken, enthält der Trinkwasserzusatz gemäß einer bevorzugten Weiterbildung zusätzlich 1 % - 95 %, insbesondere 20 % - 92 %, eines Trägers bzw. Nährstoffes.

Gemäß einer weiteren Aufgabe zielt die vorliegende Erfindung darauf ab, durch eine gezielte Verwendung von Mikroorganismen bzw. einer Mischung von Mikroorganismen das Immunsystem von Säugern und/oder Nutzvögeln gezielt zu stärken und dadurch pathogene Keime aus dem Verdauungstrakt so vollständig wie möglich zu verdrängen.

Zur Lösung dieser Aufgaben wird gemäß der vorliegenden Erfindung eine Mischung von wenigstens zwei Mikroorganismen, gewählt aus der Gruppe Enterococcus faecium, DSM 16211, Lactobacillus reuteri, DSM 16350, und Lactobacillus salivarius ssp. salivarius, DSM 16351, Pediococcus acidilactici, DSM 16210, und Bifidobacterium animalis, DSM 16284, als probiotischer, gesundheits- bzw. leistungsfördernder Nahrungs-, Futtermittel- und/ oder Trinkwasserzusatz verwendet. Durch eine derartige Verwendung gelingt es, pathogene Keime durch die sogenannte "competitive exclusion" nahezu vollständig aus dem Verdauungstrakt von Säugern bzw. Nutzvögeln zu verdrängen, da insbesondere durch eine gezielte Mischung dieser Mikroorganismen eine extrem rasche und starke Absenkung des pH-Werts im Verdauungstrakt gelingt, wobei durch die erfindungsgemäße Verwendung pathogene Keime an ihrer Entwicklung gezielt gehindert werden können. Darüber hinaus zeigte bei einer derartigen Verwendung keiner der Mikroorganismen auch bei länger dauernder gleichzeitiger Verwendung von wenigstens einem Antibiotikum keinerlei Resistenz diesen gegenüber.

Gemäß einer bevorzugten Weiterbildung wird gemäß der vorliegenden Erfindung eine Mischung von Enterococcus faecium, DSM 16211, Lactobacillus reuteri, DSM 16350, und Lactobacillus salivarius ssp. salivarius, DSM 16351, verwendet. Durch eine derartige Verwendung gelingt es insbesondere, die Wirkung von pathogenen Keimen, nämlich E. coli, zu inhibieren und aufgrund der überaus raschen Absenkung des pH-Werts im Verdauungstrakt die Lebensbedingungen für pathogene Keime per se so weit zu verschlechtern, daß auch das Wachstum sämtlicher anderen schädlichen Keime stark behindert bzw. inhibiert ist, wodurch die Krankheitsanfälligkeit der damit versorgten Säuger und/oder Nutzvögel deutlich absinkt.

Gemäß einer Weiterbildung der Erfindung wird bevorzugt eine Mischung aus Enterococcus faecium, DSM 16211, Pediococcus acidilactici, DSM 16210, und Bifidobacterium animalis, DSM 16284, verwendet, mit welcher insbesondere die Inhibierung von Salmonella choleraesuis, Campylobacter jejuni bzw. Clostridium perfringens gelingt und überdies auch die Lebensbedingungen für weitere pathogene Keime stark verschlechtert werden, wodurch insgesamt eine probiotische Wirkung der verwendeten Mischung nachgewiesen werden kann, welche weit über die Summenwirkung der Verwendung der einzelnen Mikroorganismen hinausgeht.

Gemäß einer bevorzugten Weiterbildung der Erfindung wird eine Mischung, bestehend aus Enterococcus faecium, DSM 16211, Lactobacillus reuteri, DSM 16350, gemeinsam mit weiteren Stämmen aus diesen Gruppen Lactobacilli, Streptococci und/oder Bifidobacteria verwendet, wobei durch den zusätzlichen Einsatz von Stämmen aus diesen Gruppen Lactobacilli, Streptococci und/oder Bifidobacteria ein gezielter Einsatz bzw. eine gezielte Inhibierung von speziellen Mikroorganismen an gezielten Bereichen des Magen-Darm-Trakts möglich wird, wodurch insbesondere das Auftreten von einer speziellen Krankheit, welche durch pathogene Keime verursacht wird, inhibiert werden kann.

Gemäß einer bevorzugten Weiterbildung der Erfindung wird die Mischung aus Mikroorganismen zur Steigerung der Immunabwehr eingesetzt. Ein derartiger Einsatz wird insbesondere dadurch möglich, daß durch gezielte Auswahl der verwendeten Mikroorganismen und deren Herkunft aus den verschiedensten Bereichen des Verdauungstrakts sichergestellt werden kann, daß die verwendeten Mikroorganismen über die gesamte Länge des Verdauungssystems ihre Wirkung entfalten und somit tatsächlich die Immunabwehr des damit behandelten Säugers bzw. Nutzvogels gegenüber speziellen, pathogenen Keimen im gesamten Verdauungstrakt deutlich gesteigert werden kann.

Gemäß einer bevorzugten Weiterbildung wird die Mischung aus Mikroorganismen gemeinsam mit wenigstens einem Gel- bzw. Granulatbildner oder Coatingmaterial, gewählt aus Maltodextrinen, Guarkernmehl, Gummi arabicum, Alginaten, modifizierter Stärke und Stärkederivaten, Dextrinen, Cellulosederivaten, Proteinen, Akaziengummi, Traganthgummi, Lipiden, verwendet. Durch eine Verwendung der Mischung aus Mikroorganismen mit einem Gel- bzw. Granulatbildner gelingt eine Verwendung als sogenannte Granulat- bzw. Gelapplikation, wodurch insbesondere extrem junge Tiere, welche nicht mehr von der Mutter mit den entsprechenden immunsteigernden Substanzen versorgt werden können, sicher und zuverlässig mit den nötigen, für die Steigerung der Immunabwehr erforderlichen Mikroorganismen versorgt werden können. Bei einer derartigen Gel- bzw. Granulatapplikation wird die Mischung aus Mikroorganismen mit Gelbildnern überzogen bzw. verkapselt und diese Mischung direkt auf die damit zu versorgenden Jungtiere aufgesprüht bzw. aufgebracht oder beispielsweise bei Küken in die Transportbox eingebracht, wodurch eine Versorgung der Jungtiere mit den für sie essentiell wichtigen Mikroorganismen sichergestellt werden kann. Eine ähnliche bevorzugte Verwendung gelingt damit, daß die Mischung aus Mikroorganismen gemeinsam mit einem flüssigen Träger als Sprühsuspension direkt auf die damit zu behandelnden Jungtiere aufgebracht wird. Indem die Jungtiere mit der Mischung aus Mikroorganismen besprüht werden, wird ein Teil dieser Sprühmischung durch selbst vorgenommene Fellpflege bzw. Kontakt mit anderen Jungtieren in den Verdauungstrakt aufgenommen, wodurch wiederum eine Versorgung mit den essentiell für die Steigerung der Immunabwehr notwendigen Mikroorganismen sichergestellt ist.

Die Erfindung wird nachfolgend anhand von Ausführungsbeispielen weiter erläutert. In diesen zeigt Beispiel 1 die Inhibierung von pathogenen Keimen durch die isolierten Testkeime im Agarplatten-Testmodell, Beispiel 2 einen Fütterungsversuch, bei welchem Tagesküken mit einem Futtermittel- und einem Trinkwasserzusatz, enthaltend fünf, drei bzw. keine Mikroorganismen, versorgt wurden, Beispiel 3 einen Feldversuch, bei welchem Tagesküken mit einem Trinkwasserzusatz, enthaltend fünf Mikroorganismen, versorgt wurden, Beispiel 4 einen Fütterungsversuch, bei welchem Tagesküken zu definierten Zeitpunkten einen Trinkwasserzusatz, enthaltend fünf Mikroorganismen, erhielten, Beispiel 5 Salmonella-Challenge bei Tagesküken, welche Mikroorganismen entweder über das Trinkwasser oder über das Futter zu definierten Zeitpunkten verabreicht wurden, wobei eine Mischung aus drei bzw. fünf Mikroorganismen zum Einsatz gelangte, und Beispiel 6 einen Fütterungsversuch bei Absetzferkeln.

### Beispiel 1

### Inhibierung von pathogenen Keimen durch die erfindungsgemäßen, isolierten "CE-Testkeime" ("competitive exclusion"-Testkeime) im Agarplatten-Testmodell

Der Versuch wurde auf MRS-Agarplatten durchgeführt. Die Stämme wurden mittels Impfen auf die Agarplatten aufgebracht, wobei die Stämme direkt aus den Kühlphiolen entnommen wurden. Die Inkubation der Platten erfolgte für 48 h unter anaeroben Bedingungen. Nach Anzucht der pathogenen Stämme in den entsprechenden Medien wurden die Platten mit etwa 9 ml des halbfesten Mediums, welches den pathogenen Stamm enthält, vorsichtig übergossen. Für E. coli und Salmonella chloeraesuis wurde halbfestes VL-Medium, für Campylobacter jejuni halbfestes "Brain Heart Infusion Medium" und für Clostridium perfringens halbfestes "Reinforced Clostridial Medium" verwendet.

Die Platten wurden anschließend unter den jeweiligen Inkubationsbedingungen der pathogenen Stämme inkubiert. Die Auswertung der Platten erfolgte durch Ausmessen des Durchmessers der Testkeime und der Hemmhöfe und daraus die Berechnung des Verhältnisses Hemmhof zu Testkeim. Aus den Verhältnissen Hemmhof/Testkeim kann eine Bewertung der inhibierenden Wirkung der Testkeime durchgeführt werden. In der nachfolgenden Tabelle 1 sind die Ergebnisse für die verwendeten Stämme allein und in Mischung angeführt.

**Tabelle 1**

| Isolat | Stamm | E.coli | E.coli | Salmonella choleraesuis | Campylobacter jejuni | Clostridium perfringens |
|---|---|---|---|---|---|---|
| DSM 16211 | Enterococcus faecium | 2,11 | 1,44 | 1,37 | 1,28 | 1,43 |
| DSM 16284 | Bifidobacterium animalis | 0,45 | 0,85 | 0,84 | 0,33 | 1,85 |
| DSM 16210 | Pediococcus acidilactici | 1.52 | 1,38 | 1,34 | 1,47 | 1,51 |
| DSM 16351 | Lactobacillus salivarius | 1,49 | 2,26 | 1,46 | 1,50 | 2,05 |
| DSM 16350 | Lactobacillus reuteri | 2,53 | 1,42 | 1,60 | 1,36 | 2,12 |
| Bereich | | 0,45 - 2,5 | 0,85 - 2,26 | 0.64 - 1,37 | 0,33 - 1,5 | 1,01 - 2,12 |
| *NCIMB 10415* | *Enterococcus faecium* | *1,37* | *1,35* | *1,20* | *1,22* | *1,31* |
| *DSM 20104* | *Bifidobacterium animalis* | *0,41* | *0,79* | *0,65* | *0,31* | *1,42* |
| *DSM 20284* | *Pediococcus acidilactici* | *1,41* | *1,20* | *1,10* | *1,32* | *1,35* |
| *DSM 20555* | *Lactobacillus salivarius* | *1,40* | *2,19* | *1.09* | *1,41* | *1,63* |
| *DSM 20016* | *Lactobacillus reuteri* | *2,14* | *1,30* | *1,20* | *1,35* | *1,54* |
| Mittlere Wirkung der Einzelstämme | | 1,48 | 1,56 | 1,11 | 0,92 | 1,57 |
| DSM 16211, | | 1,70 | 1,70 | 1,90 | 1,30 | 1,60 |
| DSM 16210, | | | | | | |
| DSM 16284 | | | | | | |
| DSM 16211, | | 2,50 | 1,80 | 1,60 | 1,30 | 1,80 |
| DSM 16350, | | | | | | |
| DSM 16351 | | | | | | |
| DSM 16210, | | 2,60 | 1,90 | 2,10 | 1,70 | 1,90 |
| DSM 16211, | | | | | | |
| DSM 16350, | | | | | | |
| DSM 16351, | | | | | | |
| DSM 16284 | | | | | | |

Aus dem Vergleich der hinterlegten Mikroorganismen (fett geschrieben) mit verwandten Stämmen (kursiv geschrieben) ergibt sich eindeutig, daß die hinterlegten Mikroorganismen (fett geschrieben) pathogene Keime in einem bedeutend höheren Ausmaß als bereits bekannte Stämme (kursiv) hemmen können.

### Beispiel 2

Fütterungsversuch an Tagesküken, welchen entweder eine Mischung aus allen fünf hinterlegten Mikroorganismen oder eine Mischung aus drei hinterlegten Mikroorganismen verabreicht wurde, wobei in der Starterperiode die Mischungen über das Trinkwasser verabreicht wurden und zusätzlich während der gesamten Versuchsperiode über das Futter

### Versuchsparameter:

600 Tagesküken (Rasse: Ross 308) wurden in 3 Gruppen aufgeteilt:
- Gruppe 1:: Kontrolle ohne Zusätze
- Gruppe 2:: fünf Mikroorganismen; Tag 1 - 21 im Trinkwasser; Tag 1 - 42 über das Futter
- Gruppe 3:: DSM 16210, DSM 16211, DSM 16284; Tag 1 - 21 im Trinkwasser; Tag 1 - 42 über das Futter

Wasser und Futter standen den Tieren zur Aufnahme ad libitum zur Verfügung.

### Erfaßte Leistungsparameter:

Lebendgewicht, tägl. Zunahmen, Futteraufnahme, Futterverwertung (= FCR), Mortalität, EPEF (European Production Efficiency Factor)

**Tabelle 2: Zusammenfassung Leistungsdaten des Fütterungsversuches**

| Gruppe | Lebendgewicht (g) | Tägl. Zunahme (g) | Futteraufnahme (g/Tier) | FCR | Mortalität % | EPEF |
|---|---|---|---|---|---|---|
| Kontrolle | 2588^{a} | 60,7 | 91,0 | 1,68 | 6,4 | 343 |
| DSM 16210, | 2657 ^{ab} | 62,4 | 91,3 | 1,68 | 5,0 | 357 |
| DSM 16211, | | | | | | |
| DSM 16284 | | | | | | |
| DSM 16211, | 2689^{b} | 63,1 | 92,3 | 1,69 | 3,3 | 366 |
| DSM 16284, | | | | | | |
| DSM 16210, | | | | | | |
| DSM 16351, | | | | | | |
| DSM 16350 | | | | | | |

| | | | | | | |
|---|---|---|---|---|---|---|
| a, b sign. Unterschied (P < 0,05) * EPEF (European Production Efficiency Factor) | | | | | | |

Durch den Einsatz der Produkte konnte das Lebendgewicht der Tiere in den Versuchsgruppen signifikant verbessert werden. Die Futteraufnahme konnte in der Produktgruppe, welche alle fünf Mikroorganismen erhielt, leicht gesteigert werden, hinsichtlich der Futterverwertung konnte allerdings kein Unterschied festgestellt werden. Die Mortalität war allgemein hoch, da der Versuch im Hochsommer durchgeführt wurde und die Tiere mit einer sehr hohen Umgebungstemperatur konfrontiert waren. Trotz dieses zusätzlichen Streßfaktors konnte jedoch ein deutlicher Leistungsanstieg in den Produktgruppen beobachtet werden.

### Beispiel 3

In einem Feldversuch wurde eine Mischung aus allen fünf Mikroorganismen an Tagesküken unter Praxisbedingungen getestet und im Trinkwasser über die gesamte Periode verabreicht.

### Versuchsparameter:

Ca. 53.000 Tagesküken wurden gleichmäßig auf 2 Etagen aufgeteilt.

### Erdgeschoß Kontrolle ohne Zusätze

### 1. Stock alle fünf Mikroorganismen im Trinkwasser Wasser und Futter standen den Tieren ad libitum zur Verfügung.

### Erfaßte Leistungsparameter:

### Lebendgewicht, Mortalität

Die Leistungen der Tiere konnten, wie dies der nachfolgenden Tabelle 3 entnommen werden kann, deutlich gesteigert werden, vor allem auch im Vergleich zu vorangegangenen Produktionszyklen, die in Tabelle 4 aufgelistet sind, und die zuvor aufgetretenen E. coli Probleme konnten in den Griff bekommen werden.

**Tabelle 3: Zusammenfassung Leistungsdaten**

| | Lebendgewicht (g) | Mortalität % |
|---|---|---|
| Erdgeschoß | 2282 | 3,38 |
| 1. Stock | 2328 | 3,03 |

**Tabelle 4: Leistung der letzten 6 Produktionszyklen der Farm**

| | Anzahl Tiere | Mortalität (%) | Lebendgewicht (g) |
|---|---|---|---|
| 1 | 51000 | 5,55 | 2109 |
| 2 | 49990 | 6,46 | 2228 |
| 3 | 49000 | 2,65 | 2043 |
| 4 | 50592 | 5,41 | 1627 |
| 5 | 48450 | 5,09 | 1785 |
| 6* | 53244 | 3,20 | 2305 |

| | | | |
|---|---|---|---|
| * ... mit allen fünf Mikroorganismen | | | |

### Beispiel 4

In einem Fütterungsversuch wurde Tagesküken eine Mischung aus allen fünf Mikroorganismen über das Trinkwasser zu definierten Zeitpunkten verabreicht.

### Versuchsparameter:

400 Tagesküken der Rasse Ross 308 wurden nach dem Zufallsprinzip in 2 Gruppen aufgeteilt:
- Gruppe 1: Kontrolle; ohne Zusätze
- Gruppe 2: fünf Mikroorganismen im Trinkwasser am Tag 1, 2, 3, 7, 8, 9 und 14, 15, 16
Wasser und Futter standen den Tieren zur Aufnahme ad libitum zur Verfügung.

### Erfaßte Leistungsparameter

### Lebendgewichtszunahme, Futteraufnahme, Futterverwertung (= FCR), Mortalität, EPEF (European Production Efficiency Factor)

Die Ergebnisse sind in Tabelle 5 zusammengefaßt.

**Tabelle 5: Zusammenfassung Leistungsdaten**

| | Kontrolle | fünf Mikroorganismen |
|---|---|---|
| Lebendgewicht (g) | 2320,66a | 2392,19b |
| Zunahme (g) | 54,17 | 55,87 |
| Futterverbrauch (g) | 4365 | 4482 |
| Futterverwertung (FCR) | 1,92 | 1,90 |
| Mortalität (%) | 1 | 1,5 |
| EPEF* | 285 | 295 |

| | | |
|---|---|---|
| a, b sign. Unterschiede (P < 0,05) * EPEF (European Production Efficiency Factor) | | |

Durch den Zusatz der Mikroorganismen konnte das Lebendgewicht der Tiere signifikant gesteigert werden. Bei der Futteraufnahme bzw. Futterverwertung waren beide Gruppen miteinander vergleichbar. Die Produktivitätsmassezahl (= EPEF) lag in der Produktgruppe deutlich höher als in der Kontrolle.

### Beispiel 5

### Salmonella-Challenge bei Tagesküken

Ein Produkt aus allen fünf Mikroorganismen und ein Produkt, bestehend aus drei Mikroorganismen, wurde im Futter über die gesamte Versuchsperiode und über das Trinkwasser in der Starterperiode verabreicht.

### Versuchsparameter:

304 Tagesküken der Rasse Cobb wurden in 4 Gruppen aufgeteilt:
- Gruppe 1: negative Kontrolle; ohne Zusätze und ohne Salmonellen-Challenge
- Gruppe 2: positive Kontrolle; ohne Zusätze; mit Salmonellen-Challenge
- Gruppe 3: mit Salmonellen-Challenge + Produkt aus fünf Mikroorganismen: Tag 1 - 21 im Trinkwasser; Tag 142 über das Futter
- Gruppe 4: mit Salmonellen-Challenge + Produkt aus drei Mikroorganismen (DSM 16211, DSM 16210, DSM 16284): Tag 1 - 21 im Trinkwasser; Tag 1 - 42 über das Futter

Wasser und Futter standen den Tieren zur Aufnahme ad libitum zur Verfügung.

Alle Tiere der Gruppe 2, 3 und 4 wurden am fünften Tag oral mit einer Dosis von 6 x 10⁵ cfu Salmonella enteritidis inokuliert. Die negative Kontrolle wurde zur Kontrolle der normalen Leistungsdaten mitgeführt.

### Erfaßte Leistungsparameter:

Lebendgewichtszunahme, Futteraufnahme, Futterverwertung (= FCR), Morbidität (gemessen als Anzahl der Durchfälle), Mortalität, EPEF (European Production Efficiency Factor) Die Ergebnisse sind in Tabelle 6 zusammengestellt.

**Tabelle 6: Zusammenfassung Leistungsdaten**

| | 1 | 2 | 3 | 4 |
|---|---|---|---|---|
| Gesamtzunahme (g) | 2847,29^{a} | 2632,39^{b} | 2716,49^{ab} | 2702,18 |
| Futterverbrauch (g) | 4790,07^{a} | 4559,11^{b} | 4534,30^{b} | 4527,02 |
| Futterverwertung (FCR) | 1,68 | 1,73 | 1,67 | 1,67 |
| % Morbidität | 6,34^{a} | 19,14^{b} | 15,65^{b} | 16,79^{b} |
| % Mortalität | 0,96 | 0,47 | 0,72 | 0,669 |
| EPEF* | 422 | 385 | 416 | 412 |

| | | | | |
|---|---|---|---|---|
| a, b sign. Unterschiede (P < 0,05) * EPEF (European Production Efficiency Factor) | | | | |

Hinsichtlich der Gesamtzunahme kam es durch die Infektion mit Salmonella enteritidis zu deutlichen Leistungseinbußen im Vergleich zu der Kontrollgruppe, die durch den Zusatz der Produkte gemäß der Erfindung verbessert werden konnten. Es zeigte sich hiebei, daß sowohl bei der Zugabe des Produkts aus drei Mikroorganismen als auch jenem aus fünf Mikroorganismen eine deutliche Verbesserung erzielt werden konnte. Die Futteraufnahme wurde durch die Infektion ebenfalls negativ beeinflußt, allerdings konnte die Futterverwertung durch den Zusatz der erfindungsgemäßen Produkte deutlich verbessert werden. Auch die Morbidität, gemessen an der Anzahl der beobachteten Durchfälle, konnte durch den Zusatz der Produkte im Vergleich zu der nicht behandelten Gruppe deutlich reduziert werden.

### Beispiel 6

### Fütterungsversuch bei Tagesküken - Salmonella-Challenge

Je drei Gruppen bestehend aus 36 Tagesküken wurden einem Fütterungsversuch Salmonella-Challenge unterzogen. Einer ersten Gruppe wurde keinerlei Produkt verabreicht, einer zweiten Gruppe wurde eine Mischung aus allen fünf hinterlegten Stämmen verabreicht und eine dritte Gruppe erhielt ein kommerziell erhältliches probiotisches Produkt bestehend aus folgenden Mikroorganismen: Lactobacillus sp., Lactobacillus sp., Bifidobacterium sp und Enterococcus faecium.

### Versuchsansatz:

In einem isolierten Raum wurden die Tagesküken in Käfigen gehalten. Die probiotischen Produkte wurden über das Trinkwasser, das den Küken ad libitum zur Verfügung stand, verabreicht, und zwar am Lebenstag 1, 2 und 3. Am dritten Lebenstag wurde allen Küken oral 1,0 ml einer Suspension von Salmonella enteriditis verabreicht. Die Konzentration der Salmonellenlösung betrug 1,0 x 10⁶ CFU/ml. Alle Küken erhielten zusätzlich Futter und Wasser ad libitum. Gemessen wurde die Konzentration der Salmonellen im Kot der Tiere.

### Auswertung:

| Gruppe | Konzentration von Salmonellen |
|---|---|
| | im Kot (CFU log 10) |
| Kommerziell erhältliches probiotisches Produkt | 2,43^{b} |
| Produktmischung | n.d. (<2,00)^{a} |
| Kontrolle | 3,62 |

Die Ergebnisse zeigen, daß einzig die Kombination aus den fünf hinterlegten Mikroorganismen in der Lage war, das Wachstum von Salmonellen signifikant zu reduzieren, und zwar so weit, daß die Anzahl der Salmonellenkeime unter den Detektionslimit lag.

### Beispiel 7

Fütterungsversuch bei Absetzferkeln unter Einsatz einer Mischung, bestehend aus fünf Mikroorganismen, und einer Mischung, bestehend aus drei Mikroorganismen

In einem Fütterungsversuch wurde die Mikroorganismen gemäß der vorliegenden Erfindung auf ihrer Auswirkungen auf die Leistung bei Absetzferkeln getestet. Die Produkte wurde über die gesamte Versuchsperiode von 47 Tagen über das Futter verabreicht.

### Versuchsparameter:

101 Absetzferkel der Rasse ÖHYB wurden in 3 Gruppen aufgeteilt:
- Gruppe 1: Kontrolle ohne Zusätze
- Gruppe 2: fünf Mikroorganismen (C5); Tag 1 - 47 über das Futter
- Gruppe 3: drei Mikroorganismen (C3), bestehend aus DSM 16211, DSM 16350, DSM 16351; Tag 1 - 47 über das Futter

Wasser und Futter standen den Tieren zur Aufnahme ad libitum zur Verfügung.

### Erfaßte Leistungsparameter:

### Lebendgewicht, tägliche Zunahmen, Futteraufnahme, Futterverwertung (= FCR), Mortalität

Die Ergebnisse sind in Tabelle 7 zusammengefaßt.

**Tabelle 7: Zusammenfassung Leistungsdaten**

| | Kontrolle | C5 | Unterschied zw. C5 u. Kontrolle | C3 | Unterschied zw. C3 u. Kontrolle |
|---|---|---|---|---|---|
| Anzahl Tiere | 34 | 33 | | 33 | |
| Anfangsgewicht (kg) | 7,75 | 7,76 | | 7,75 | |
| Gewicht Tag 13 (kg) | 11,89 | 12,26 | + 3,1 % | 12,09 | + 1,7 % |
| Endgewicht (kg) | 31,67 | 33,00 | + 4,2 % | 32,5 | + 2,6 % |
| Tägl. Zunahme Tag 1 - 13 (g) | 321 | 350 | + 9,0 % | 334 | + 4,0 % |
| Tägl. Zunahme Tag 14 - 47 (g) | 582 | 610 | + 4,8 % | 600 | + 3,09 % |
| Tägl. Zunahme Tag 1 - 47 (g) | 509 | 537 | + 5,5 % | 527 | + 3,5 % |
| Mortalität (Anzahl) | 2 | 2 | | | |

Durch den Einsatz der Produkte konnte das Lebendgewicht der Tiere in den Versuchsgruppen deutlich, nämlich um 3 % bzw. 1,7 % nach den ersten 13 Tagen und um 4 % bzw. 2,6 % bis zum Versuchsende, verbessert werden. Auch bei den täglichen Zunahmen konnte eine deutliche Steigerung durch den Zusatz der Produkte erreicht werden.

In der nachfolgenden Tabelle 8 sind die Futterdaten zusammengefaßt.

**Tabelle 8: Zusammenfassung Futterdaten**

| | kontrolle | C5 | unterschied | C3 | Unterschied |
|---|---|---|---|---|---|
| Futterverbrauch (g) | | | | | |
| Tag 1 - 13 | 495 | 511 | + 3,2 % | 504 | + 1,8 |
| Tag 14 - 47 | 1155 | 1193 | + 3,3 % | 1180 | + 2,2 |
| Tag 1 - 47 | 972 | 1033 | + 6,3 | 1012 | + 4,1 |

| FCR (kg/kg) | | | | | |
|---|---|---|---|---|---|
| Tag 1 - 13 | 1,76 | 1,66 | | 1,68 | |
| Tag 14 - 47 | 1,98 | 2,02 | | 2,00 | |
| Tag 1 - 47 | 1,95 | 1,96 | | 1,95 | |

Die Futteraufnahme konnte in den Produktgruppen leicht gesteigert werden, hinsichtlich Futterverwertung ergab sich vor allem in der ersten Versuchsperiode eine Verbesserung. Im weiteren Verlauf konnte allerdings kein Unterschied in der Futterverwertung festgestellt werden.

Liste gemäß Regel 13bis, Absatz 4 der Ausführungsordnung zum Vertrag über die internationale Zusammenarbeit auf dem Gebiet des Patentwesens

Sämtliche der in der vorliegenden Anmeldung genannten Mikroorganismen wurden bei der DSMZ - Deutsche Sammlung von Mikroorganismen und Zellkulturen GmbH, Mascheroder Weg 1b, 38124 Braunschweig, Deutschland (DE), hinterlegt.

| Eingangsnummer | Eingangsdatum |
|---|---|
| DSM 16284 | 10.03.2004 |
| DSM 16211 | 06.02.2004 |
| DSM 16210 | 06.02.2004 |
| DSM 16350 | 15.04.2004 |
| DSM 16351 | 15.04.2004 |

### Beispiel 8

### Positive Verschiebung der Darmflora bei Masthühnern

Vier Gruppen von Masthühnern wurden einem Fütterungsversuch unterzogen, wobei 2 Gruppen eine Produktmischung, bestehend aus fünf hinterlegten Mikroorganismen, verabreicht wurde, eine Gruppe ist eine Negativkontrolle ohne jede Zusätze, eine Gruppe eine Positivkontrolle unter Zusatz eines antibiotischen Leistungsförderes, nämlich Avialmycin, eine Gruppe, die Mischung aus fünf Mikroorganismen über das Trinkwasser verabreicht bekam und eine Gruppe, die Mischung von Mikroorganismen über das Futter verabreicht bekam.

Im Zuge der Überprüfung stellte sich heraus, daß es zwischen den einzelnen Gruppen keine statistisch signifikanten Unterschiede in bezug auf aerobe Gesamtkeimzahl, Coliforme, anaerobe Gesamtkeimzahl und Bacteroide gab. Jene Gruppen, die die Mischung aus fünf Mikroorganismen verabreicht bekamen, hatten im Vergleich zur Positivkontrolle und Negativkontrolle (NK, PK) signifikant höhere Keimzahlen in bezug auf auf Bifidobacterium sp, Lactobacillus spp und Gram+Kokken (z.B. Enterococcus, Pediococcdus).

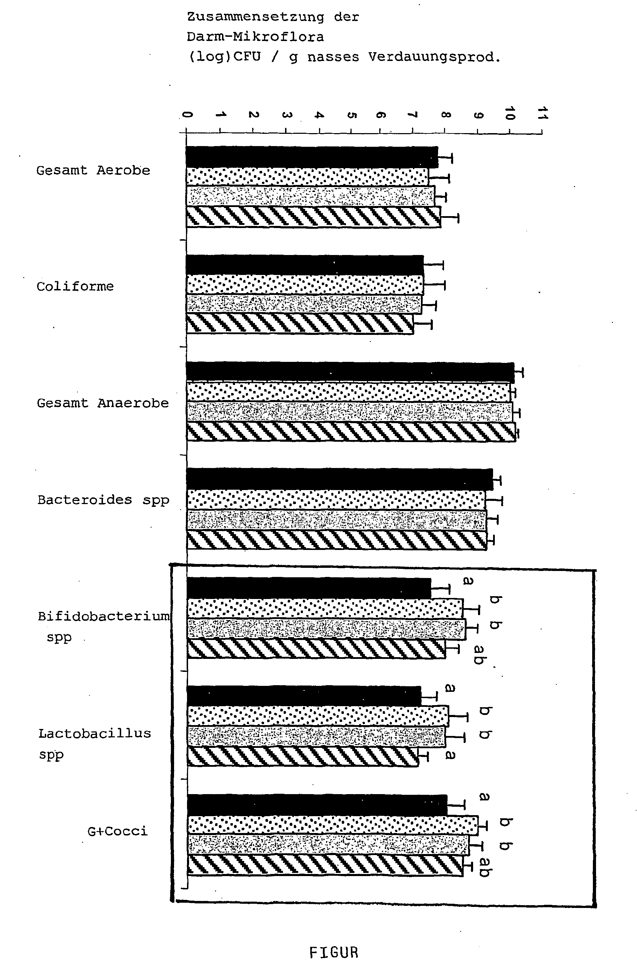

In der Figur ist ein Flußdiagramm dieses Vergleichs dargestellt.

In dem Flußdiagramm der Figur kann in dem schwarz umrahmten Teil gezeigt werden, daß jene Gruppen, die die Mischung aus fünf Mikroorganismen über das Trinkwasser bzw. das Futter verabreicht bekamen, eine bedeutend höhere Anzahl von "guten" Keimen aufwiesen. In dem Diagramm der Figur ist die Gruppe der Negativkontrolle als schwarzer Balken dargestellt, die Gruppe der Positivkontrolle als schraffierter Balken, die Gruppe, die die fünf Mikroorganismen über das Futtermittel verabreicht bekamen, als gepunkteter Balken, und jene Gruppe, die die Mischung aus fünf Mikroorganismen über das Trinkwasser verabreicht bekamen, als grauer einfärbiger Balken dargestellt.

### Beispiel 9

### Fütterungsversuch bei Masthühnern

4500 gemischtgeschlechtliche Masthühner (Ross 308) wurden in zwei Gruppen aufgeteilt, wobei jede der beiden Gruppen in weitere drei Untergruppen zur Vereinfachung der Beobachtung unterteilt wurde. Beide Gruppen erhielten ein Standardfuttermittel und die Testgruppe erhielt eine Mischung aus den fünf hinterlegten Mikroorganismen in einer Dosierung von 1*10⁹ CFU/kg Futter. Die Kontrollgruppe erhielt ein kommerziell erhältliches probiotisches Produkt in der gleichen Dosierung. Die Verabreichung der Substanzen erfolgte über das Futtermittel.

Nach 38 Tagen konnte festgestellt werden, daß die Leistungsparameter der Testgruppe deutlich besser waren und zwar sowohl in bezug auf das Lebendgewicht, die täglichen Zunahmen und die Futterverwertung. Darüber hinaus ist die Mortalität gegenüber der Vergleichsgruppe deutlich abgesunken. Die Ergebnisse sind in der nachfolgenden Tabelle 9 gezeigt.

**Tabelle 9**

| Ergebnisse nach 38 Tagen | | |
|---|---|---|
| | Kommerziell erhältliches Probiotisches Produkt | C5-Produktmischung |
| Anzahl der Tiere | 2250 | 2250 |
| Lebendgewicht(g) | 1875 | 2034 |
| Tägliche Zunahmen (g) | 52 | 56,5 |
| Futterverwertung | 1,66 | 1,61 |
| Mortalität (%) | 1,15 | 0,40 |

### Beispiel 10

In einem Laborversuch wurden Antibiotikaresistenzen der fünf hinterlegten Mikroorganismen untersucht und soferne verfügbar, mit den Antibiogrammen von verwandten, Difidobakterien, verglichen. Lediglich beim Stamm Bifidobakterium animalis konnte kein Vergleichsstamm in der Literatur gefunden werden bzw. aufgrund von schwierigen Anzuchtbedingungen gesicherte Daten für Vergleichsstämme erhalten werden. Die Ergebnisse der Resistenzversuche gegenüber gängigen Antibiotika zusammen mit soweit vorhandenen Vergleichsstämmen sind in den nachfolgenden Tabellen gezeigt.

| | Pediococcus acidilacti, DSM 16210 | Pediococcus acidilacti, Referenzstamm |
|---|---|---|
| **β-Lactame -** Ampicillin | S | |
| **Aminoglycodside** | | |
| Streptomycin | S | R |
| Kanamycin | S | R |
| Neomycin | S | R |
| Gentamicin | S | R |
| **Amphenicol -** Chloramphenicol | S | S |
| **Macrolid -** Erythromycin | S | S |
| **Ansamycin -** Rifampin | S | S |
| **Streptogramin -** Chinu/dalfopristin | S | R |
| Fluorochinolon - Enrofloxacin | S | n.a. |
| **Oxazolidion-** Linezolid | S | R |

| | Enterococcus faecium, DSM 16211 | Enterococcus faecium, Referenzstramm 1 | Enterococcus faecium, Referenzstramm 2 |
|---|---|---|---|
| **β-Lactame -** Ampicillin | S | S | S |
| **Aminoglycoside** | | | |
| Streptomycin | S | R | S |
| Kanamycin | S | R | R |
| Neomycin | S | R | R |
| Gentamicin | S | R | S |
| **Amphenicol -** Chloramphenicol | S | S | S |
| **Tetracyclin** | S | R | S |
| **Macrolide -** Erythromycin | S | R | R |
| **Ansamycin -** Rifampin | S | S | S |
| **Streptogramin-** Chinu/dalfopristin | S | R | n.a. |
| **Oxazolidion-** Linezolid | S | S | n.a. |
| **Folat inhibitor-** Trimethoprim | S | R | n.a. |
| **Glycopeptid -** Vancomycin | S | S | S |

| | Lactobacillus saliv. ssp. Saliv., DSM 16351 | Lactobacillus salivarius, Referenzstamm 1 | Lactobacillus salivarius, Referenzstamm 2 |
|---|---|---|---|
| **β-Lactame -** Ampicillin | S | S | S |
| **Amphenicol -** Chloramphenicol | S | S | S |
| **Tetracyclin** | S | S | R |
| **Macrolid -** Erythromycin | S | S | S |
| **Ansamycin -** Rifampin | S | S | S |
| **Streptogramin -** Chinu/dalfopristin | S | R | S |
| **Oxazolidion-** Linezolid | S | S | S |
| **Folat inhibitor -** Trimethoprim | S | R | R |

| | Lactobacillus reuteri, DSM 16350 | Lactobacillus reuteri, Referenzstamm |
|---|---|---|
| **Amphenicol -** Chloramphenicol | S | S |
| **Mcrolid -** Erythromycin | S | R |
| **Ansamycin -** Rifampin | S | S |
| **Streptogramin -** Chinu/dalfopristin | S | R |
| **Oxazolidion-** Linezolid | S | S |

| | Bifidobacterium animalis, DSM 16284 | |
|---|---|---|
| **β-Lactame** - Ampicillin | S | |
| Aminoglycoside | | |
| Neomycin | S | |
| Gentamicin | S | |
| **Amphenicol** - Chloramphenicol | S | |
| **Tetraciclin** | S | |
| **Macrolid** - Erythromycin | S | |
| **Ansamycin** - Rifampin | S | |
| **Strptogramin** - Chinu/dalfopristin | S | |
| **Fluorochinolon**- Enrofloxacin | S | |
| **Oxazolidion-** Linezolid | S | |
| **Folat inhibitor -** Trimethoprim | S | |
| **Glycopeptid-** Vancomycin | S | |

| | | |
|---|---|---|
| S ... sensitive, R ... resistent | | |

Aus diesen Vergleichsversuchen ergibt, daß keiner der hinterlegten Mikroorganismen gegen eines der bekannten getesteten Antibiotika resistent wurde, sie blieben alle sensitiv bzw. aufnahmefähig.

### Anlage zu PCT-Anmeldung

Anmelder: Erber Aktiengesellschaft et al.

Liste gemäß Regel 13bis, Absatz 4 der Ausführungsordnung zum Vertrag über die internationale Zusammenarbeit auf dem Gebiet des Patentwesens

Die in Anspruch 1 in der vorliegenden PCT-Anmeldung genannten Mikroorganismen wurden bei der DSMZ - Deutsche Sammlung von Mikroorganismen und Zellkulturen GmbH, Mascheroder Weg 1b, 38124 Braunschweig, Deutschland (DE), hinterlegt.

| Eingangsnummer | Eingangsdatum |
|---|---|
| DSM 16284 | 10.03.2004 |
| DSM 16211 | 06.02.2004 |
| DSM 16210 | 06.02.2004 |
| DSM 16350 | 15.04.2004 |
| DSM 16351 | 15.04.2004 |

## Patentansprüche

1. Probiotischer, gesundheits- bzw. leistungsfördernder Nahrungs-, Futtermittel- und/oder Trinkwasserzusatz, welcher eine Mischung von Mikroorganismen enthält, **dadurch gekennzeichnet, daß** wenigstens zwei Mikroorganismen, gewählt aus der Gruppe Enterococcus faecium, DSM 16211, Lactobacillus reuteri, DSM 16350, und Lactobacillus salivarius ssp. salivarius, DSM 16351, Pediococcus acidilactici, DSM 16210, und Bifidobacterium animalis, DSM 16284, enthalten sind.

2. Nahrungs-, Futtermittel- und/oder Trinkwasserzusatz nach Anspruch 1, **dadurch gekennzeichnet, daß** wenigstens drei Mikroorganismen enthalten sind.

3. Nahrungs-, Futtermittel- und/oder Trinkwasserzusatz nach Anspruch 1 oder 2, **dadurch gekennzeichnet, daß** Enterococcus faecium, DSM 16211, Lactobacillus reuteri, DSM 16350, und Lactobacillus sälivarius ssp. salivarius, DSM 16351, enthalten sind.

4. Nahrungs-, Futtermittel- und/oder Trinkwasserzusatz nach Anspruch 1 oder 2, **dadurch gekennzeichnet, daß** Enterococcus faecium, DSM 16211, Pediococcus acidilactici, DSM 16210, und Bifidobacterium animalis, DSM 16284, enthalten sind.

5. Nahrungs-, Futtermittel- und/oder Trinkwasserzusatz nach einem der Ansprüche 1 bis 4, **dadurch gekennzeichnet, daß** zusätzlich als Träger einsetzbare Nährsubstanzen und/oder prebiotische Substanzen, gewählt aus Fructo-Oligosacchariden, Inulinen, Isomalto-Oligosacchariden, Lactitol, Lactosucrose, Lactulose, Pyrodextrinen, Soja-Oligosacchariden, Transgalacto-Oligosacchariden, Xylo-Oligosaccardien, Vitaminen, insbesondere Vitamin E, enthalten sind.

6. Nahrungs-, Futtermittel- und/oder Trinkwasserzusatz nach einem der Ansprüche 1 bis 5, **dadurch gekennzeichnet, daß** ein weiterer Träger, gewählt aus Zeolithen, Calciumkarbonat, Magnesiumkarbonat, Trehalose, Chitosan, Schellack, Albumin, Stärke, Magermilchpulver, Süßmolkenpulver, Maltodextrine, Lactose, Inulin, Dextrosen, pflanzlichen Ölen, oder ein Lösungsmittel, gewählt aus Wasser oder physiologischer Kochsalzlösung, enthalten ist.

7. Nahrungs-, Futtermittel- und/oder Trinkwasserzusatz nach einem der Ansprüche 1 bis 6, **dadurch gekennzeichnet, daß** zusätzlich ein Beschichtungsmaterial, gewählt aus Maltodextrinen, Guarkernmehl, Gummi arabicum, Alginaten, modifizierter Stärke und Stärkederivaten, Dextrinen, Cellulosederivaten, wie Celluloseester und -ether, Proteinen, wie Gelatine, Albumin, Casein, Gluten, Akaziengummi, Traganthgummi, Lipiden, wie Wachsen, Paraffin, Stearinsäure, Mono- und Diglyceriden, enthalten ist.

8. Nahrungs-, Futtermittel- und/oder Trinkwasserzusatz nach einem der Ansprüche 1 bis 7, **dadurch gekennzeichnet, daß** zusätzlich wenigstens ein Mikroorganismus, gewählt aus der Gruppe Bifidobacterium sp., Lactobacillus salivarius, Lactobacillus sp., Lactobacillus fermentum und Enterococcus faecalis enthalten ist.

9. Nahrungs-, Futtermittel- und/oder Trinkwasserzusatz nach einem der Ansprüche 1 bis 8, **dadurch gekennzeichnet, daß** die Mikroorganismen in suspendierter, pulverförmiger oder verkapselter Form mit einem maximalen Durchmesser von 2000 µm enthalten sind.

10. Futtermittelzusatz, insbesondere für Nutzvögel und/oder Schweine zur Leistungssteigerung der Nutztiere, nach einem der Ansprüche 1 bis 9, **dadurch gekennzeichnet, daß** der Futtermittelzusatz aus 1 x 10⁷ bis 1 x 10¹⁴, insbesondere 1 x 10¹⁰ bis 1 x 10¹², KBE/kg Futtermittelzusatz Enterococcus faecium, DSM 16211, und 1 x 10⁶ bis 1 x 10¹⁴, insbesondere 1 x 10¹⁰ bis 1 x 10¹², KBE/kg Futtermittelzusatz Lactobacillus reuteri, DSM 16350, besteht.

11. Futtermittelzusatz, insbesondere für Nutzvögel und/oder Schweine zur Leistungssteigerung der Nutztiere, nach einem der Ansprüche 1 bis 9, **dadurch gekennzeichnet, daß** 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁷ bis 1 x 10¹⁴, insbesondere 1 x 10¹⁰ bis 1 x 10¹², KBE/kg Futtermittelzusatz Pediococcus acidilactici, DSM 16210, und 1 x 10⁷ bis 1 x 10¹⁴, insbesondere 1 x 10¹⁰ bis 1 x 10¹², KBE/kg Futtermittelzusatz Bifidobacterium animalis, DSM 16284, enthalten sind.

12. Futtermittelzusatz, insbesondere für Nutzvögel und/oder Schweine zur Leistungssteigerung der Nutztiere, nach einem der Ansprüche 1 bis 9, **dadurch gekennzeichnet, daß** der Futtermittelzusatz aus 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁶ bis 1 x 10¹⁴, insbesondere 1 x 10¹⁰ bis 1 x 10¹², KBE/kg Futtermittelzusatz Lactobacillus reuteri, DSM 16350, und 1 x 10⁶ bis 1 x 10¹⁴, insbesondere 1 x 10¹⁰ bis 1 x 10¹², KBE/kg Futtermittelzusatz Lactobacillus salivarius ssp. salivarius, DSM 16351, besteht.

13. Futtermittelzusatz, insbesondere für Nutzvögel und/oder Schweine zur Leistungssteigerung der Nutztiere, nach einem der Ansprüche 1 bis 9, **dadurch gekennzeichnet, daß** der Futtermittelzusatz aus 1 x 10⁷ bis 1 x 10¹⁴, insbesondere 1 x 10¹⁰ bis 1 x 10¹², KBE/kg Futtermittelzusatz Enterococcus faecium, DSM 16211, 1 x 10⁶ bis 1 x 10¹⁴, insbesondere 1 x 10¹⁰ bis 1 x 10¹², KBE/kg Futtermittelzusatz Lactobacillus reuteri, DSM 16350, 1 x 10⁶ bis 1 x 10¹⁴, insbesondere 1 x 10¹⁰ bis 1 x 10¹², KBE/kg Futtermittelzusatz Lactobacillus salivarius ssp. salivarius, DSM 16351, 1 x 10⁷ bis 1 x 10¹⁴, insbesondere 1 x 10¹⁰ bis 1 x 10¹², KBE/kg Futtermittelzusatz Pediococcus acidilactici, DSM 16210, 1 x 10⁷ bis 1 x 10¹⁴, insbesondere 1 x 10¹⁰ bis 1 x 10¹², KBE/kg Futtermittelzusatz Bifidobacterium animalis, DSM 16284, und 1 x 10⁶ bis 1 x 10¹⁴, insbesondere 1 x 10¹⁰ bis 1 x 10¹² KBE/kg Futtermittelzusatz Bifidobacterium sp. 2 besteht.

14. Futtermittelzusatz nach Anspruch 11, 12 oder 13, **dadurch gekennzeichnet, daß** der Futtermittelzusatz zusätzlich 1 % - 95 %, insbesondere 20 % - 98 %, eines Trägers bzw. Naturstoffes enthält.

15. Futtermittelzusatz nach Anspruch 11, 12 oder 13, **dadurch gekennzeichnet, daß** der Futtermittelzusatz als wäßrige Suspension in einer Menge von 0,01 g/kg - 10 g/kg, insbesondere 0,025 g/kg bis 2,5 g/kg, Futtermittel auf das Futtermittel aufgebracht ist.

16. Futtermittelzusatz nach einem der Ansprüche 10 bis 15, **dadurch gekennzeichnet, daß** der Futtermittelzusatz in einer Menge von 20 g/t bis 20 kg /t, insbesondere 100 g/t bis 2,5 kg/t, in das Futtermittel eingemischt ist.

17. Trinkwasserzusatz, insbesondere für Nutzvögel und/oder Schweine zur Leistungssteigerung der Nutztiere, nach einem der Ansprüche 1 bis 9, **dadurch gekennzeichnet, daß** der Trinkwasserzusatz 1 x 10⁷ bis 1 x 10¹⁴, insbesondere 1 x 10¹⁰ bis 1 x 10¹³, KBE/kg Trinkwasserzusatz Enterococcus faecium, DSM 16211, und 1 x 10⁶ bis 1 x 10¹⁴, insbesondere 1 x 10¹⁰ bis 1 x 10¹³, KBE/kg Trinkwasserzusatz Lactobacillus reuteri, DSM 16350, enthält.

18. Trinkwasserzusatz, insbesondere für Nutzvögel und/oder Schweine zur Leistungssteigerung der Nutztiere, nach einem der Ansprüche 1 bis 9, **dadurch gekennzeichnet, daß** der Trinkwasserzusatz aus 1 x 10⁷ bis 1 x 10¹⁴, insbesondere 1 x 10¹⁰ bis 1 x 10¹³, KBE/kg Trinkwasserzusatz Enterococcus faecium, DSM 16211, 1 x 10⁶ bis 1 x 10¹⁴, insbesondere 1 x 10¹⁰ bis 1 x 10¹³, KBE/kg Trinkwasserzusatz Lactobacillus reuteri, DSM 16350, und 1 x 10⁶ bis 1 x 10¹⁴, insbesondere 1 x 10¹⁰ bis 1 x 10¹³, KBE/kg Trinkwasserzusatz Lactobacillus salivarius ssp. salivarius, DSM 16351, besteht.

19. Trinkwasserzusatz, insbesondere für Nutzvögel und/oder Schweine zur Leistungssteigerung der Nutztiere, nach einem der Ansprüche 1 bis 9, **dadurch gekennzeichnet, daß** der Trinkwasserzusatz aus 1 x 10⁷ bis 1 x 10¹⁴, insbesondere 1 x 10¹⁰ bis 1 x 10¹³, KBE/kg Trinkwasserzusatz Enterococcus faecium, DSM 16211, 1 x 10⁷ bis 1 x 10¹⁴, insbesondere 1 x 10¹⁰ bis 1 x 10¹³, KBE/kg Trinkwasserzusatz Pediococcus acidilactici, DSM 16210, und 1 x 10⁷ bis 1 x 10¹⁴, insbesondere 1 x 10¹⁰ bis 1 x 10¹³, KBE/kg Trinkwasserzusatz Bifidobacterium animalis, DSM 16284, besteht.

20. Trinkwasserzusatz, insbesondere für Nutzvögel und/oder Schweine zur Leistungssteigerung der Nutztiere, nach einem der Ansprüche 1 bis 9, **dadurch gekennzeichnet, daß** der Trinkwasserzusatz aus 1 x 10⁷ bis 1 x 10¹⁴, insbesondere 1 x 10¹⁰ bis 1 x 10¹³, KBE/ Trinkwasser Enterococcus faecium, DSM 16211, 1 x 10⁶ bis 1 x 10¹⁴, insbesondere 1 x 10¹⁰ bis 1 x 10¹³, KBE/kg Trinkwasserzusatz Lactobacillus reuteri, DSM 16350, 1 x 10⁶ bis 1 x 10¹⁴, insbesondere 1 x 10¹⁰ bis 1 x 10¹³, KBE/kg Trinkwasserzusatz Lactobacillus salivarius ssp. salivarius, DSM 16351, 1 x 10⁷ bis 1 x 10¹⁴, insbesondere 1 x 10¹⁰ bis 1 x 10¹³, KBE/kg Trinkwasserzusatz Pediococcus acidilactici, DSM 16210, 1 x 10⁷ bis 1 x 10¹⁴, insbesondere 1 x 10¹⁰ bis 1 x 10¹³, KBE/kg Trinkwasserzusatz Bifidobacterium animalis, DSM 16284, und 1 x 10⁶ bis 1 x 10¹⁴, insbesondere 1 x 10¹⁰ bis 1 x 10¹³ KBE/kg Trinkwasserzusatz Bifidobacterium sp. 2 besteht.

21. Trinkwasserzusatz nach einem der Ansprüche 17 bis 20, **dadurch gekennzeichnet, daß** der Trinkwasserzusatz zusätzlich 1 % - 95 %, insbesondere 20 % - 92 %, eines Trägers bzw. Nährstoffes enthält.

22. Verwendung einer Mischung von wenigstens zwei Mikroorganismen, gewählt aus der Gruppe Enterococcus faecium, DSM 16211, Lactobacillus reuteri, DSM 16350, und Lactobacillus salivarius ssp. salivarius, DSM 16351, Pediococcus acidilactici, DSM 16210, und Bifidobacterium animalis, DSM 16284, zur Herstellung eines probiotischen, gesundheits-bzw. leistungsfördernden Nahrungs-, Futtermittel- und/ oder Trink-wasserzusatzes.

23. Verwendung nach Anspruch 22, **dadurch gekennzeichnet, daß** Enterococcus faecium, DSM 16211, Lactobacillus reuteri, DSM 16350, und Lactobacillus salivarius ssp. salivarius, DSM 16351, eingesetzt werden.

24. Verwendung nach Anspruch 22, **dadurch gekennzeichnet, daß** Enterococcus faecium, DSM 16211, Pediococcus acidilactici, DSM 16210, und Bifidobacterium animalis, DSM 16284, eingesetzt werden.

25. Verwendung nach Anspruch 22, **dadurch gekennzeichnet, daß** Enterococcus faecium, DSM 16211, Lactobacillus reuteri, DSM 16350, gemeinsam mit weiteren Stämmen aus diesen Gruppen Lactobacilli, Streptococci und/oder Bifidobacteria eingesetzt werden.

26. Verwendung nach einem der Ansprüche 22 bis 25, **dadurch gekennzeichnet, daß** die Mischung aus Mikroorganismen zur Steigerung der Immunabwehr eingesetzt wird.

27. Verwendung nach einem der Ansprüche 22 bis 26, **dadurch gekennzeichnet, daß** die Mischung aus Mikroorganismen gemeinsam mit wenigstens einem Gelbildner, gewählt aus Maltodextrinen, Guarkernmehl, Gummi arabicum, Alginaten, modifizierter Stärke und Stärkederivaten, Dextrinen, Cellulosederivaten, Proteinen, Akaziengummi, Traganthgummi, Lipiden, eingesetzt wird.

28. Verwendung nach einem der Ansprüche 22 bis 27, **dadurch gekennzeichnet, daß** die Mischung aus Mikroorganismen gemeinsam mit einem flüssigen Träger als Sprühsuspension eingesetzt wird.

## Claims

1. A probiotic health and performance promoting food, feed and/or drinking water additive containing a mixture of microorganisms, **characterized in that** it comprises at least two microorganisms selected from the group consisting of Enterococcus faecium, DSM 16211, Lactobacillus reuteri, DSM 16350, and Lactobacillus salivarius ssp. salivarius, DSM 16351, Pediococcus acidilactici, DSM 16210, and Bifidobacterium animalis, DSM 16284.

2. A food, feed and/or drinking water additive according to claim 1, **characterized in that** it comprises at least three microorganisms.

3. A food, feed and/or drinking water additive according to claim 1 or 2, **characterized in that** it comprises Enterococcus faecium, DSM 16211, Lactobacillus reuteri, DSM 16350, and Lactobacillus salivarius ssp. salivarius, DSM 16351.

4. A food, feed and/or drinking water additive according to claim 1 or 2, **characterized in that** it comprises Enterococcus faecium, DSM 16211, Pediococcus acidilactici, DSM 16210, and Bifidobacterium animalis, DSM 16284.

5. A food, feed and/or drinking water additive according to any one of claims 1 to 4, **characterized in that** it comprises nutritive substances and/or prebiotic substances additionally usable as carriers and selected from fructo-oligosaccharides, inulins, isomalto-oligosaccharides, lactitol, lactosucrose, lactulose, pyrodextrines, soy oligosaccharides, transgalacto-oligosaccharides, xylo-oligosaccharides, vitamins, in particular vitamin E.

6. A food, feed and/or drinking water additive according to any one of claims 1 to 5, **characterized in that** it comprises a further carrier selected from zeolites, calcium carbonate, magnesium carbonate, trehalose, chitosan, shellac, albumin, starch, skim-milk powder, swee-whey powder, maltodextrin, lactose, inulin, dextroses, vegetable oils, or a solvent selected from water or physiologic saline solution.

7. A food, feed and/or drinking water additive according to any one of claims 1 to 6, **characterized in that** it additionally comprises a coating material selected from maltodextrins, guar seed flour, gum arabic, alginates, modified starch and starch derivates, dextrins, cellulose derivates like cellulose ester and ether, proteins like gelatine, albumin, casein, gluten, acacia gum, tragacanth, lipids like waxes, paraffin, stearic acid, mono- and diglycerides.

8. A food, feed and/or drinking water additive according to any one of claims 1 to 7, **characterized in that** it additionally comprises at least one microorganism selected from the group of Bifidobacterium sp., Lactobacillus salivarius, Lactobacillus sp., Lactobacillus fermentum and Enterococcus faecalis.

9. A food, feed and/or drinking water additive according to any one of claims 1 to 8, **characterized in that** the microorganisms are contained in suspended, powdery or encapsulated form with a maximum diameter of 2000 µm.

10. A feed additive, in particular for farm birds and/or pigs to increase the performance of said farm animals, according to any one of claims 1 to 9, **characterized in that** said feed additive is comprised of 1 x 10⁷ to 1 x 10¹⁴, in particular 1 x 10¹⁰ to 1 x 10¹², CFU/kg feed additive Enterococcus faecium, DSM 16211, and 1 x 10⁶ to 1 x 10¹⁴, in particular 1 x 10¹⁰ to 1 x 10¹², CFU/kg feed additive Lactobacillus reuteri, DSM 16350.

11. A feed additive, in particular for farm birds and/or pigs to increase the performance of said farm animals, according to any one of claims 1 to 9, **characterized in that** said feed additive is comprised of 1 x 10⁷ to 1 x 10¹⁴, in particular 1 x 10¹⁰ to 1 x 10¹², CFU/kg feed additive Enterococcus faecium, DSM 16211, 1 x 10⁷ to 1 x 10¹⁴, in particular 1 x 10¹⁰ to 1 x 10¹², CFU/kg feed additive Pediococcus acidilactici, DSM 16210, and 1 x 10⁷ to 1 x 10¹⁴, in particular 1 x 10¹⁰ to 1 x 10¹², CFU/kg feed additive Bifidobacterium animalis, DSM 16284.

12. A feed additive, in particular for farm birds and/or pigs to increase the performance of said farm animals, according to any one of claims 1 to 9, **characterized in that** said feed additive is comprised of 1 x 10⁷ to 1 x 10¹⁴, in particular 1 x 10¹⁰ to 1 x 10¹², CFU/kg feed additive Enterococcus faecium, DSM 16211, 1 x 10⁶ to 1 x 10¹⁴, in particular 1 x 10¹⁰ to 1 x 10¹², CFU/kg feed additive Lactobacillus reuteri, DSM 16350, and 1 x 10⁶ to 1 x 10¹⁴, in particular 1 x 10¹⁰ to 1 x 10¹², CFU/kg feed additive Lactobacillus salivarius ssp. salivarius, DSM 16351.

13. A feed additive, in particular for farm birds and/or pigs to increase the performance of said farm animals, according to any one of claims 1 to 9, **characterized in that** said feed additive is comprised of 1 x 10⁷ to 1 x 10¹⁴, in particular 1 x 10¹⁰ to 1 x 10¹², CFU/kg feed additive Enterococcus faecium, DSM 16211, 1 x 10⁶ to 1 x 10¹⁴, in particular 1 x 10¹⁰ to 1 x 10¹², CFU/kg feed additive Lactobacillus reuteri, DSM 16350, 1 x 10⁶ to 1 x 10¹⁴, in particular 1 x 10¹⁰ to 1 x 10¹², CFU/kg feed additive Lactobacillus salivarius ssp. salivarius, DSM 16351, 1 x 10⁷ to 1 x 10¹⁴, in particular 1 x 10¹⁰ to 1 x 10¹², CFU/kg feed additive Pediococcus acidilactici, DSM 16210, 1 x 10⁷ to 1 x 10¹⁴, in particular 1 x 10¹⁰ to 1 x 10¹², CFU/kg feed additive Bifidobacterium animalis, DSM 16284, and 1 x 10⁶ to 1 x 10¹⁴, in particular 1 x 10¹⁰ to 1 x 10¹² CFU/kg, feed additive Bifidobacterium sp. 2.

14. A feed additive according to claim 11, 12 or 13, **characterized in that** the feed additive additionally comprises 1% to 95%, in particular 20% to 98%, of a carrier or natural substance.

15. A feed additive according to claim 11, 12 or 13, **characterized in that** the feed additive is applied on the feed as an aqueous suspension in an amount of from 0.01 g/kg to 10 g/kg feed, in particular 0.025 g/kg to 2.5 g/kg feed.

16. A feed additive according to any one of claims 10 to 15, **characterized in that** the feed additive is mixed into the feed in an amount of from 20 g/t to 20 kg/t, in particular 100 g/t to 2.5 kg/t.

17. A drinking water additive, in particular for farm birds and/or pigs to increase the performance of said farm animals, according to any one of claims 1 to 9, **characterized in that** the drinking water additive comprises 1 x 10⁷ to 1 x 10¹⁴, in particular 1 x 10¹⁰ to 1 x 10¹³, CFU/kg drinking water additive Enterococcus faecium, DSM 16211, and 1 x 10⁶ to 1 x 10¹⁴, in particular 1 x 10¹⁰ to 1 x 10¹³, CFU/kg drinking water additive Lactobacillus reuteri, DSM 16350.

18. A drinking water additive, in particular for farm birds and/or pigs to increase the performance of said farm animals, according to any one of claims 1 to 9, **characterized in that** the drinking water additive is comprised of 1 x 10⁷ to 1 x 10¹⁴, in particular 1 x 10¹⁰ to 1 x 10¹³, CFU/kg drinking water additive Enterococcus faecium, DSM 16211, 1 x 10⁶ to 1 x 10¹⁴, in particular 1 x 10¹⁰ to 1 x 10¹³, CFU/kg drinking water additive Lactobacillus reuteri, DSM 16350, and 1 x 10⁶ to 1 x 10¹⁴, in particular 1 x 10¹⁰ to 1 x 10¹³, CFU/kg drinking water additive Lactobacillus salivarius ssp. salivarius, DSM 16351.

19. A drinking water additive, in particular for farm birds and/or pigs to increase the performance of said farm animals, according to any one of claims 1 to 9, **characterized in that** the drinking water additive is comprised of 1 x 10⁷ to 1 x 10¹⁴, in particular 1 x 10¹⁰ to 1 x 10¹³, CFU/kg drinking water additive Enterococcus faecium, DSM 16211, 1 x 10⁷ to 1 x 10¹⁴, in particular 1 x 10¹⁰ to 1 x 10¹³, CFU/kg drinking water additive Pediococcus acidilactici, DSM 16210, and 1 x 10⁷ to 1 x 10¹⁴, in particular 1 x 10¹⁰ to 1 x 10¹³, CFU/kg drinking water additive Bifidobacterium animalis, DSM 16284.

20. A drinking water additive, in particular for farm birds and/or pigs to increase the performance of said farm animals, according to any one of claims 1 to 9, **characterized in that** the drinking water additive is comprised of 1 x 10⁷ to 1 x 10¹⁴, in particular 1 x 10¹⁰ to 1 x 10¹³, CFU/kg drinking water additive Enterococcus faecium, DSM 16211, 1 x 10⁶ to 1 x 10¹⁴, in particular 1 x 10¹⁰ to 1 x 10¹³, CFU/kg drinking water additive Lactobacillus reuteri, DSM 16350, 1 x 10⁶ to 1 x 10¹⁴, in particular 1 x 10¹⁰ to 1 x 10¹³, CFU/kg drinking water additive Lactobacillus salivarius ssp. Salivarius, DSM 16351, 1 x 10⁷ to 1 x 10¹⁴, in particular 1 x 10¹⁰ to 1 x 10¹³, CFU/kg drinking water additive Pediococcus acidilactici, DSM 16210, 1 x 10⁷ to 1 x 10¹⁴, in particular 1 x 10¹⁰ to 1 x 10¹³, CFU/kg drinking water additive Bifidobacterium animalis, DSM 16284, and 1 x 10⁶ to 1 x 10¹⁴, in particular 1 x 10¹⁰ to 1 x 10¹³ CFU/kg drinking water additive Bifidobacterium sp. 2.

21. A drinking water additive according to any one of claims 17 to 20, **characterized in that** the drinking water additive additionally comprises 1% to 95%, in particular 20% to 92%, of a carrier or nutrient.

22. The use of a mixture of at least two microorganisms selected from the group consisting of Enterococcus faecium, DSM 16211, Lactobacillus reuteri, DSM 16350, and Lactobacillus salivarius ssp. salivarius, DSM 16351, Pediococcus acidilactici, DSM 16210, and Bifidobacterium animalis, DSM 16284, for the production of a probiotic health and performance promoting food, feed and/or drinking water additive.

23. The use according to claim 22, **characterized in that** Enterococcus faecium, DSM 16211, Lactobacillus reuteri, DSM 16350, and Lactobacillus salivarius ssp. salivarius, DSM 16351, are used.

24. The use according to claim 22, **characterized in that** Enterococcus faecium, DSM 16211, Pediococcus acidilactici, DSM 16210, and Bifidobacterium animalis, DSM 16284, are used.

25. The use according to claim 22, **characterized in that** Enterococcus faecium, DSM 16211, Lactobacillus reuteri, DSM 16350, are used along with other strains from these groups of Lactobacilli, Streptococci and/or Bifidobacteria.

26. The use according to any one of claims 22 to 25, **characterized in that** the mixture of microorganisms is used to increase immune defence.

27. The use according to any one of claims 22 to 26, **characterized in that** the mixture of microorganisms is used along with at least one gelatinizing agent selected from maltodextrins, guar seed flour, gum arabic, alginates, modified starch and starch derivates, dextrins, cellulose derivates, proteins, acacia gum, tragacanth, lipids.

28. The use according to any one of claims 22 to 27, **characterized in that** the mixture of microorganisms, along with a liquid carrier, is used as a spray suspension.

## Revendications

1. Additif alimentaire, d'alimentation animale et/ou pour eau potable, probiotique, bon pour la santé ou renforçant les performances, qui contient un mélange de microorganismes, **caractérisé en ce qu'**au moins deux microorganismes choisis dans le groupe Enterococcus faecium, DSM 16211, Lactobacillus reuteri, DSM 16350 et Lactobacillus salivarius ssp. salivarius, DSM 16351, Pediococcus acidilactici, DSM 16210 et Bifidobacterium animalis, DSM 16284 sont contenus.

2. Additif alimentaire, d'alimentation animale et/ou pour eau potable selon la revendication 1, **caractérisé en ce qu'**au moins trois microorganismes sont contenus.

3. Additif alimentaire, d'alimentation animale et/ou pour eau potable selon la revendication 1 ou 2, **caractérisé en ce que** Enterococcus faecium, DSM 16211, Lactobacillus reuteri, DSM 16350 et Lactobacillus salivarius ssp. salivarius, DSM 16351 sont contenus.

4. Additif alimentaire, d'alimentation animale et/ou pour eau potable selon la revendication 1 ou 2, **caractérisé en ce que** Enterococcus faecium, DSM 16211, Pediococcus acidilactici, DSM 16210 et Bifidobacterium animalis, DSM 16284 sont contenus.

5. Additif alimentaire, d'alimentation animale et/ou pour eau potable selon l'une quelconque des revendications 1 à 4, **caractérisé en ce que**, en outre, des substances nutritives utilisables comme support et/ou des substances prébiotiques choisies parmi les fructo-oligosaccharides, les inulines, les isomalto-oligosaccharides, le lactitol, le lactosaccharose, le lactulose, les pyrodextrines, les oligosaccharides de soja, les transgalacto-oligosaccharides, les xylo-oligosaccharides, les vitamines, en particulier la vitamine E sont contenus.

6. Additif alimentaire, d'alimentation animale et/ou pour eau potable selon l'une quelconque des revendications 1 à 5, **caractérisé en ce qu'**un autre support choisi parmi les zéolithes, le carbonate de calcium, le carbonate de magnésium, le tréhalose, le chitosan, la gomme laque, l'albumine, l'amidon, la poudre de lait écrémé, la poudre de petit lait, les maltodextrines, le lactose, l'inuline, les dextroses, les huiles végétales ou un solvant choisi parmi l'eau ou une solution physiologique est contenu.

7. Additif alimentaire, d'alimentation animale et/ou pour eau potable selon l'une quelconque des revendications 1 à 6, **caractérisé en ce qu'**en outre un matériau de revêtement choisi parmi les maltodextrines, la farine de graines de guar, la gomme arabique, les alginates, l'amidon modifié et les dérivés d'amidon, les dextrines, les dérivés de cellulose tels que l'ester de cellulose et l'éther de cellulose, les protéines telles que la gélatine, l'albumine, la caséine, le gluten, la gomme arabique, la gomme adragante, les lipides tels que les cires, la paraffine, l'acide stéarique, les mono- et diglycérides est contenu.

8. Additif alimentaire, d'alimentation animale et/ou pour eau potable selon l'une quelconque des revendications 1 à 7, **caractérisé en ce que qu**'en outre au moins un micro-organisme choisi dans le groupe Bifidobacterium sp., Lactobacillus salivarius, Lactobacillus sp., Lactobacillus fermentum et Enterococcus faecalis est contenu.

9. Additif alimentaire, d'alimentation animale et/ou pour eau potable selon l'une quelconque des revendications 1 à 8, **caractérisé en ce que** les microorganismes sont contenus sous forme en suspension, en poudre ou encapsulée présentant un diamètre maximal de 2000 µm.

10. Additif d'alimentation animale, en particulier pour les oiseaux d'élevage et/ou les cochons pour augmenter la performance des animaux d'élevage, selon l'une quelconque des revendications 1 à 9, **caractérisé en ce que** l'additif d'alimentation animale est constitué de 1 x 10⁷ à 1 x 10¹⁴, en particulier 1 x 10¹⁰ à 1 x 10¹² UFC/kg d'additif d'alimentation animale Enterococcus faecium, DSM 16211 et 1 x 10⁶ à 1 x 10¹⁴, en particulier 1 x 10¹⁰ à 1 x 10¹² UFC/kg d'additif d'alimentation animale de Lactobacillus reuteri, DSM 16350.

11. Additif d'alimentation animale, en particulier pour les oiseaux d'élevage et/ou les cochons pour augmenter la performance des animaux d'élevage, selon l'une quelconque des revendications 1 à 9, **caractérisé en ce que** 1 x 10⁷ à 1 x 10¹⁴, en particulier 1 x 10¹⁰ à 1 x 10¹² UFC/kg d'additif d'alimentation animale Enterococcus faecium, DSM 16211, 1 x 10⁷ à 1 x 10¹⁴, en particulier 1 x 10¹⁰ à 1 x 10¹² d'UFC/kg d'additif d'alimentation animale de Pediococcus acidilactici, DSM 16210 et 1 x 10⁷ à 1 x 10¹⁴, en particulier 1 x 10¹⁰ à 1 x 10¹² UFC/kg d'additif d'alimentation animale de Bifidobacterium animalis, DSM 16284 sont contenus.

12. Additif d'alimentation animale, en particulier pour les oiseaux d'élevage et/ou les cochons pour augmenter la performance des animaux d'élevage, selon l'une quelconque des revendications 1 à 9, **caractérisé en ce que** l'additif d'alimentation animale est constitué de 1 x 10⁷ à 1 x 10¹⁴, en particulier 1 x 10¹⁰ à 1 x 10¹² UFC/kg d'additif d'alimentation animale Enterococcus faecium, DSM 16211, 1 x 10⁶ à 1 x 10¹⁴, en particulier 1 x 10¹⁰ à 1 x 10¹² UFC/kg d'additif d'alimentation animale Lactobacillus reuteri, DSM 16350 et 1 x 10⁶ à 1 x 10¹⁴, en particulier 1 x 10¹⁰ à 1 x 10¹² UFC/kg d'additif d'alimentation animale Lactobacillus salivarius ssp. salivarius, DSM 16351.

13. Additif d'alimentation animale, en particulier pour les oiseaux d'élevage et/ou les cochons pour augmenter la performance des animaux d'élevage, selon l'une quelconque des revendications 1 à 9, **caractérisé en ce que** l'additif d'alimentation animale est constitué de 1 x 10⁷ à 1 x 10¹⁴, en particulier 1 x 10¹⁰ à 1 x 10¹² UFC/kg d'additif d'alimentation animale Enterococcus faecium, DSM 16211, 1 x 10⁶ à 1 x 10¹⁴, en particulier 1 x 10¹⁰ à 1 x 10¹² UFC/kg d'additif d'alimentation animale Lactobacillus reuteri, DSM 16350, 1 x 10⁶ à 1 x 10¹⁴, en particulier 1 x 10¹⁰ à 1 x 10¹² UFC/kg d'additif d'alimentation animale Lactobacillus salivarius ssp. salivarius, DSM 16351, 1 x 10⁷ à 1 x 10¹⁴, en particulier 1 x 10¹⁰ à 1 x 10¹² UFC/kg d'additif d'alimentation animale Pediococcus acidilactici, DSM 16210, 1 x 10⁷ à 1 x 10¹⁴, en particulier 1 x 10¹⁰ à 1 x 10¹² UFC/kg d'additif d'alimentation animale Bifidobacterium animalis, DSM 16284, et 1 x 10⁶ à 1 x 10¹⁴, en particulier 1 x 10¹⁰ à 1 x 10¹² UFC/kg d'additif d'alimentation animale Bifidobacterium sp. 2.

14. Additif d'alimentation animale selon la revendication 11, 12 ou 13, **caractérisé en ce que** l'additif d'alimentation animale contient en outre 1 % à 95 %, en particulier 20 % à 98 % d'un support ou d'une substance naturelle.

15. Additif d'alimentation animale selon la revendication 11, 12 ou 13, **caractérisé en ce que** l'additif d'alimentation animale est ajouté en tant que suspension aqueuse en une quantité de 0,01 g/kg à 10 g/kg, en particulier 0,025 g/kg à 2,5 g/kg d'aliment animal sur l'aliment animal.

16. Additif d'alimentation animale selon l'une quelconque des revendications 10 à 15, **caractérisé en ce que** l'additif d'alimentation animale est mélangé en une quantité de 20 g/t à 20 kg/t, en particulier 100 g/t à 2,5 kg/t dans l'aliment animal.

17. Additif pour eau potable, en particulier pour les oiseaux d'élevage et/ou les cochons pour augmenter la performance des animaux d'élevage, selon l'une quelconque des revendications 1 à 9, **caractérisé en ce que** l'additif pour eau potable contient 1 x 10⁷ à 1 x 10¹⁴, en particulier 1 x 10¹⁰ à 1 x 10¹³ UFC/kg d'additif pour eau potable Enterococcus faecium, DSM 16211, 1 x 10⁶ à 1 x 10¹⁴, en particulier 1 x 10¹⁰ à 1 x 10¹³ UFC/kg d'additif pour eau potable Lactobacillus reuteri, DSM 16350.

18. Additif pour eau potable, en particulier pour les oiseaux d'élevage et/ou les cochons pour augmenter la performance des animaux d'élevage, selon l'une quelconque des revendications 1 à 9, **caractérisé en ce que** l'additif pour eau potable est constitué de 1 x 10⁷ à 1 x 10¹⁴, en particulier 1 x 10¹⁰ à 1 x 10¹³ UFC/kg d'additif pour eau potable Enterococcus faecium, DSM 16211, 1 x 10⁶ à 1 x 10¹⁴, en particulier 1 x 10¹⁰ à 1 x 10¹³ UFC/kg d'additif pour eau potable Lactobacillus reuteri, DSM 16350 et 1 x 10⁶ à 1 x 10¹⁴ en particulier, 1 x 10¹⁰ à 1 x 10¹³ UFC/kg d'additif pour eau potable Lactobacillus salivarius ssp. salivarius, DSM 16351.

19. Additif pour eau potable, en particulier pour les oiseaux d'élevage et/ou les cochons pour augmenter la performance des animaux d'élevage, selon l'une quelconque des revendications 1 à 9, **caractérisé en ce que** l'additif pour eau potable est constitué de 1 x 10⁷ à 1 x 10¹⁴, en particulier 1 x 10¹⁰ à 1 x 10¹³ UFC/kg d'additif pour eau potable Enterococcus faecium, DSM 16211, 1 x 10⁷ à 1 x 10¹⁴, en particulier 1 x 10¹⁰ à 1 x 10¹³ UFC/kg d'additif pour eau potable Pediococcus acidilactici, DSM 16210 et 1 x 10⁷ à 1 x 10¹⁴ en particulier, 1 x 10¹⁰ à 1 x 10¹³ UFC/kg d'additif pour eau potable Bifidobacterium animalis, DSM 16284.

20. Additif pour eau potable, en particulier pour les oiseaux d'élevage et/ou les cochons pour augmenter la performance des animaux d'élevage, selon l'une quelconque des revendications 1 à 9, **caractérisé en ce que** l'additif pour eau potable est constitué de 1 x 10⁷ à 1 x 10¹⁴, en particulier 1 x 10¹⁰ à 1 x 10¹³ UFC/kg d'additif pour eau potable Enterococcus faecium, DSM 16211, 1 x 10⁶ à 1 x 10¹⁴, en particulier 1 x 10¹⁰ à 1 x 10¹³ UFC/kg d'additif pour eau potable Lactobacillus reuteri, DSM 16350, 1 x 10⁶ à 1 x 10¹⁴ en particulier, 1 x 10¹⁰ à 1 x 10¹³ UFC/kg d'additif pour eau potable Lactobacillus salivarius ssp. salivarius, DSM 16351, 1 x 10⁷ à 1 x 10¹⁴, en particulier 1 x 10¹⁰ à 1 x 10¹³ UFC/kg d'additif pour eau potable Pediococcus acidilactici, DSM 16211, 1 x 10⁷ à 1 x 10¹⁴, en particulier 1 x 10¹⁰ à 1 x 10¹³ UFC/kg d'additif pour eau potable Bifidobacterium animalis, DSM 16284, 1 x 10⁶ à 1 x 10¹⁴ en particulier, 1 x 10¹⁰ à 1 x 10¹³ UFC/kg d'additif pour eau potable Bifidobacterium sp. 2.

21. Additif pour eau potable selon l'une quelconque des revendications 17 à 20, **caractérisé en ce que** l'additif pour eau potable contient en outre 1 % à 95 %, en particulier 20 % à 92 % d'un support ou d'une substance nutritive.

22. Utilisation d'un mélange d'au moins deux microorganismes choisis parmi le groupe Enterococcus faecium, DSM 16211, Lactobacillus reuteri, DSM 16350 et Lactobacillus salivarius ssp. salivarius, DSM 16351, Pediococcus acidilactici, DSM 16210 et Bifidobacterium animalis, DSM 16284 pour la production d'un additif alimentaire, d'alimentation animale et/ou pour eau potable, probiotique, bon pour la santé ou renforçant les performances.

23. Utilisation selon la revendication 22, **caractérisée en ce que** l'on utilise Enterococcus faecium, DSM 16211, Lactobacillus reuteri, DSM 16350 et Lactobacillus salivarius ssp. salivarius, DSM 16351.

24. Utilisation selon la revendication 22, **caractérisée en ce que** l'on utilise Enterococcus faecium, DSM 16211, Pediococcus acidilactici, DSM 16210 et Bifidobacterium animalis, DSM 16284.

25. Utilisation selon la revendication 22, **caractérisée en ce que** l'on utilise Enterococcus faecium, DSM 16211, Lactobacillus reuteri, DSM 16350 conjointement avec d'autres souches de ces groupes Lactobacilli, Streptococci et/ou bifidobactéries.

26. Utilisation selon l'une quelconque des revendications 22 à 25, **caractérisée en ce que** le mélange de microorganismes est utilisé pour augmenter les défenses immunitaires.

27. Utilisation selon l'une quelconque des revendications 22 à 25, **caractérisée en ce que** le mélange de microorganismes est utilisé conjointement avec au moins un agent formant un gel choisi parmi les maltodextrines, la farine de graines de guar, la gomme arabique, les alginates, l'amidon modifié et les dérivés d'amidon, les dextrines, les dérivés de cellulose, les protéines, la gomme arabique, la gomme adragante, les lipides.

28. Utilisation selon l'une quelconque des revendications 22 à 27, **caractérisée en ce que** le mélange de microorganismes est utilisé conjointement avec un support liquide comme suspension de pulvérisation.
